# EUROPEAN PATENT SPECIFICATION

(11) **EP 1 305 419 B1**
(45) Date of publication and mention of the grant of the patent: **27.06.2007**
(21) Application number: 01952260.6
(22) Date of filing: 27.06.2001
(51) Int. Cl.: C12N 15/24, C12N 15/63, C07K 14/54, G01N 33/50

(54) **MODULATION BY IL-TIF/INTERLEUKIN-21**
MODULIERUNG DURCH IL-TIF/INTERLEUKIN-21
MODULATION PAR IL-TIF/INTERLEUKINE-21

(30) Priority: 27.07.2000 US 626617
(43) Date of publication of application: 02.05.2003
(60) Divisional application: 07002409.6
(73) Proprietor: Wyeth, Collegeville, PA 19426 (US)
(72) Inventor: DUMOUTIER, Laure, B-1200 Brussels (BE); RENAULD, Jean-Christophe, B-1200 Brussels (BE)
(74) Representative: Brasnett, Adrian Hugh
(86) International application number: PCT/US2001/020485
(87) International publication number: WO 2002/010393

(56) References cited:
- WO-A-00/24758
- US-A- 5 843 697
- DUMOUTIER L ET AL: "CLONING AND CHARACTERIZATION OF IL-10-RELATED T CELL-DERIVED INDUCIBLE BY IL-9" IMMUNOLOGY, BLACKWELL SCIENTIFIC PUBLICATIONS, GB, vol. 164, 2000, pages 1814-1819, XP000946156 ISSN: 0019-2805
- KOTENKO S V ET AL: "IDENTIFICATION AND FUNCTIONAL CHARACTERIZATION OF A SECOND CHAIN OF THE INTERLEUKIN-10 RECEPTOR COMPLEX" EMBO JOURNAL, OXFORD UNIVERSITY PRESS, SURREY, GB, vol. 16, no. 19, 1997, pages 5894-5903, XP002046438 ISSN: 0261-4189
- SPENCER S D ET AL: "The orphan receptor CRF2-4 is an essential subunit of the interleukin 10 receptor" JOURNAL OF EXPERIMENTAL MEDICINE, TOKYO, JP, vol. 187, no. 4, 16 February 1998 (1998-02-16), pages 571-578, XP002235139 ISSN: 0022-1007
- KOTENKO SERGEI V ET AL: "Identification of the functional interleukin-22 (IL-22) receptor complex. The IL-10R2 chain (IL-10Rbeta) is a common chain of both the IL-10 and IL-22 (IL-10-related T cell-derived inducible factor, IL-TIF) receptor complexes" JOURNAL OF BIOLOGICAL CHEMISTRY, AMERICAN SOCIETY OF BIOLOGICAL CHEMISTS, BALTIMORE, MD, US, vol. 276, no. 4, 26 January 2001 (2001-01-26), pages 2725-2732, XP002186669 ISSN: 0021-9258
- DUMOUTIER L ET AL: "Cloning and characterization of IL-22 binding protein, a natural antagonist of IL-10-related T cell-derived inducible factor/IL-22" JOURNAL OF IMMUNOLOGY, THE WILLIAMS AND WILKINS CO. BALTIMORE, US, vol. 166, no. 12, 15 June 2001 (2001-06-15), pages 7090-7095, XP002206182 ISSN: 0022-1767
- DUMOUTIER L ET AL: "HUMAN INTERLEUKIN-10-RELATED T CELL-DERIVED INDUCIBLE FACTOR: MOLECULAR CLONING AND FUNCTIONAL CHARACTERIZATION AS AN HEPATOCYTE-STIMULATING FACTOR" PROCEEDINGS OF THE NATIONAL ACADEMY OF SCIENCES OF USA, NATIONAL ACADEMY OF SCIENCE. WASHINGTON, US, vol. 97, no. 18, 29 August 2000 (2000-08-29), pages 10144-10149, XP000946025 ISSN: 0027-8424
- XIE M-H ET AL: "Interleukin (IL)-22, a novel human cytokine that signals through the interferon receptor-related proteins CRF2-4 and IL-22R" JOURNAL OF BIOLOGICAL CHEMISTRY, AMERICAN SOCIETY OF BIOLOGICAL CHEMISTS, BALTIMORE, MD, US, vol. 275, no. 40, 6 October 2000 (2000-10-06), pages 31335-31339, XP002164307 ISSN: 0021-9258

## Description

### FIELD OF THE INVENTION

This invention relates to the use of IL-TIF/IL-21 (now known as IL-22). The nucleic acid molecules are shown to be upregulated by the cytokine interleukin-9 ("IL-9"). Also disclosed are the proteins encoded thereby. These molecules have been described as "T Cell Derived Inducible Factors" or "TIFs"; however, as of now, they are known as "interleukin-21" or "IL-21", as well as "IL-TIF." For this reason, the terms will be used interchangeable herein. These nucleic acid molecules encode proteins which induce STAT activation in cells. They can be used, for example, in the stimulation of regeneration of targeted tissues . Further, their inhibitors or antagonists can be used to retard, prevent or inhibit differentiation of other tissues.

### BACKGROUND

The last decade has seen knowledge of the immune system and its regulation expand tremendously. One area of particular interest has been that of research on the proteins and glycoproteins which regulate the immune system. One of the best known families of these molecules are the cytokines. These are molecules which are involved in the "communication" of cells with each other. The individual members of the cytokine family have been found to be involved in a wide variety of pathological conditions, such as cancer and allergies. Whereas sometimes the cytokines are involved in the pathology of the condition, they are also known as being therapeutically useful.

Interleukins are one type of cytokine. The literature on interleukins is vast. An exemplary, but by no means exhaustive listing of the patents in this area includes U.S. Patent No. 4,778,879 to Mertelsmann et al.; U.S. Patent No. 4,490,289 to Stern; U.S. Patent No. 4,518,584 to Mark et al.; and U.S. Patent No. 4,851,512 to Miyaji et al., all of which involve interleukin-2 or "IL-2." Additional patents have issued which relate to interleukin-1 ("IL-1"), such as U.S. Patent No. 4,808,611 to Cosman. More recent patents on different interleukins include U.S. Patent Nos. 5,694,234 (IL-13); 5,650,492 (IL-12); 5,700,664, 5,371,193 and 5,215,895 (IL-11); 5,728,377, 5,710,251, 5,328,989 (IL-10); 5,580,753, 5,587,302, 5,157,112, 5,208,218 (IL-9); 5,194,375, 4,965,195 (IL-7); 5,723,120, 5,178,856 (IL-6), and 5,017,691 (IL-4). Even a cursory review of this patent literature shows the diversity of the properties of the members of the interleukin family. One can assume that the larger cytokine family shows even more diversity. See, e.g., Aggarwal et al., ed., Human Cytokines: Handbook For Basic And Clinical Research (Blackwell Scientific Publications, 1992), Paul, ed., Fundamental Immunology (Raven Press, 1993), pg 763-836, "T-Cell Derived Cytokines And Their Receptors", and "Proinflammatory Cytokines and Immunity."

The relationships between various cytokines are complex. As will be seen from the references cited herein, as the level of a particular cytokine increases or decreases, this can affect the levels of other molecules produced by a subject, either directly or indirectly. Among the affected molecules are other cytokines.

The lymphokine IL-9, previously referred to as "P40," is a T cell derived molecule which was originally identified as a factor which sustained permanent antigen independent growth of T4 cell lines. See, e.g., Uyttenhove et al., Proc. Natl. Acad. Sci. 85: 6934 (1988), and Van Snick et al., J. Exp. Med. 169: 363 (1989), and Simpson et al., Eur. J. Biochem. 183: 715 (1989).

The activity of IL-9 was at first observed on restricted T4 cell lines, failing to show activity on CTLs or freshly isolated T cells. See, e.g., Uyttenhove et al., supra, and Schmitt et al., Eur. J. Immunol. 19: 2167 (1989). This range of activity was expanded when experiments showed that IL-9 and the molecule referred to as T cell growth Factor HI ("TCGF III") are identical to MEA (Mast Cell Growth Enhancing Activity), a factor which potentiates the proliferative response of bone marrow derived mast cells to IL-3, as is described by Hültner et al., Eur. J. Immunol. and in U.S. Patent No. 5,164,317. It was also found that the human form of IL-9 stimulates proliferation of megakaryoblastic leukemia. See Yang et al., Blood 74: 1880 (1989). Work on IL-9 has shown that it also supports erythroid colony formation (Donahue et al., Blood 75(12): 2271-2275 (6-15-90)); promotes the proliferation of myeloid erythroid burst formation (Williams et al., Blood 76: 306-311 (9-1-90); and supports clonal maturation of BFU-E's of adult and fetal origin (Holbrook et al., Blood 77(10): 2129-2134 (5-15-91)). Expression of IL-9 has also been implicated in Hodgkins's disease and large cell anaplastic lymphoma (Merz et al., Blood 78(8): 1311-1317 (9-1-90). Genetic analyses of mice that were susceptible or resistant to the development of bronchial hyperresponsiveness have unraveled a linkage with the IL-9 gene as well as a correlation between IL-9 production and susceptibility in this model (Nicolaides et al., Proc. Natl. Acad. Sci. USA, 94, 13175-13180, 1997). Human genetic studies also point to the IL-9 and IL-9R genes as candidates for asthma (Doull et al., Am. J. Respir. Crit. Care Med., 153, 1280-1284, 1996; Holroyd et al., Genomics 52, 233-235, 1998). Also, IL-9 transgenic mice allowed for the demonstration that increased IL-9 expression result in lung mastocytosis, hypereosinophilia, bronchial hyperresponsiveness and high levels of IgE (Temann et al., J. Exp. Med. 188, 1307-1320, 1998; Godfraind et al., J. Immunol. 160, 3989-3996, 1998; McLane et al., Am. J. Resp. Cell. Mol. 19:713-720 (1999). Taken together, these observations strongly suggest that IL-9 plays a major role in this disease Additional work has implicated IL-9 and muteins of this cytokine in asthma and allergies. See, e.g. WO 9708321 (Levitt, et al), and WO 9824904 (Levitt, et al).

IL-9 is known to affect the levels of other molecules in subjects. See Louahed et al., J. Immunol. 154: 5061-5070 (1995; Demoulin et al., Mol. Cell. Biol. 16: 4710-4716 (1996), both incorporated by reference. It will be recognized that the molecules affected have their own functions in biological systems. For example, Demoulin et al. show that many of the known activities of IL-9 are mediated by activation of STAT transcription factors. As such, there is continued interest in trying to identify molecules whose presence and/or level is affected by other molecules, such as cytokines.

WO 00 24 758 and Dumoutier et al (J. Immunol. 2000, 164, 1814-1819) disclose IL-TIF/IL-21 polypeptide and polynucleotides encoding it and describe the activation of STAT factors by IL-TIF/IL-21.

The disclosure which follows describes such molecules. It was found that nucleic acid molecules encoding the proteins of the invention were expressed in the presence of IL-9, but not in its absence. Hence, these molecules are, inter alia, "markers" for the expression or effect of IL-9 in a subject. The molecules once referred to as T Cell Derived Inducible Factors or "TIFs" have now been referred to as interleukin-21, or "IL-21." These and other features of the invention will be seen in the disclosure which follows.

### DETAILED DESCRIPTION OF PREFERRED EMBODIMENTS

### Example 1

The murine lymphoma cell line BW5147 is well known as a cell line which can be grown in vitro, without the need to add any cytokines to its culture medium. In order to identify genes induced by IL-9, samples of BW5147 were cultured either with (200 U/ml), or without IL-9, for 24 hours. Then, total RNA was isolated, using guanidium isothiocyanate lysis, and CsCl gradient centrifugation. These techniques are well known in the art. Following this, polyadenylated RNA was purified from the total RNA, by using an oligo(dT) cellulose column. The isolated, polyA RNA was then used to generate double stranded cDNA. A commercially available oligo(dT) primer was used. Anywhere from 3-5 µg of polyA RNA were heated to 70°C for 10 minutes with 1µg of oligo (dT), and then incubated with 5x first strand buffer (250 mM HCl (pH 8.3), 375 mM KCl, 15 mM MgCl₂), 10 mM dithiothreitol, 500 µM of deoxynucleotide triphosphates, and 800 U of reverse transcriptase. Total volume of the reaction mixture was 20 µl, and the reaction was allowed to proceed at 37°C for one hour. This resulted in synthesis of the first strand of cDNA. Second strand synthesis was accomplished by adding 30µl of 5 second strand buffer (100mM Tris-HCl (pH 6.9)), 450mM KCl, 23mM MgCl₂, 0.75mM β-NAD⁺, 50mM (NH₄)₂SO₄, together with 60U of E. coli derived DNA polymerase I, 2U of E. coli RNase H, 10 U of E. coli DNA ligase, and 250 µM of deoxynucleotide triphosphates, and brought to a final volume of 150 µl. The mixture was incubated for two hours, at 16°C.

The product was extracted using phenol-chloroform, and was precipitated with ethanol. The final cDNA product was then resuspended in 200 µl of TE.

These steps were carried out for both the stimulated BW5147 cells ("tester" hereafter), and for parallel, unstimulated BW5147 cells ("driver" hereafter).

### Example 2

The cDNA prepared in Example 1 was then subjected to subtraction cloning in accordance with well known methods. To do this, six oligonucleotides were prepared:
5'-AGCACTCTCC AGCCTCTCAC CGCA-3 (SEQ ID NO: 1);
5'-GATCTGCGGT GA-3' (SEQ ID NO: 2);
5'-ACCGACGTCG ACTATCCATG AACA-3' (SEQ ID NO: 3);
5'-GATCTGTTCA TG-3' (SEQ ID NO: 4);
5'-AGGCAACTGT GCTATCCGAG GGAA-3' (SEQ ID NO: 5); and
5'-GATCTTCCCT CG-3' (SEQ ID NO: 6).

These were used as explained herein. Double stranded cDNA (2 µg), was digested with restriction endonuclease DpnII, extracted with phenol-chloroform, precipitated with ethanol, and resuspended in 20 µl of TE (10mM Tris-HCl (pH 7.5); 1mM EDTA). Twelve µl (1.2 µg), of cut cDNA was ligated to double stranded SEQ ID NOS: 1 and 2, in a mixture which included 4 µl of desalted SEQ ID NO: 1 (2mg/ml), 4 µl desalted SEQ ID NO: 2 (1 mg/ml), 10µl of 5X adapter buffer (330mM Tris-HCl, pH 7.6, 50mM MgCl₂, 5mM ATP), 7µl DTT (100mM), and 28µl of H₂O). The oligonucleotides were annealed to each other and to the sample DNA by heating the mixture to 50°C and then cooling it to 10 °C over one hour, followed by adding 5µl of T4 DNA ligase, and incubation for 12-14 hours, at 12-16°C. The mixtures were diluted by adding 140 µl of TE. PCR was then carried out on 200 µl samples, as described infra.

### Example 3

To carry out PCR, 200 µl samples containing 2 µl of the ligation product in a buffer of 66 mM Tris-HCl, pH 8.8, 4 mM MgCl₂, 16 mM (NH₄)₂SO₄, 33 µg/ml BSA, 0.3 mM of each dNTP (concentration: 500 µM), and 2 µg of SEQ ID NO: 1 were first heated at 72°C for three minutes to remove any of SEQ ID NO: 2 which was hybridized to the product of Example 2. The 3' ends were then filled in by using 5U of Taq polymerase (5 minutes, 72°C). Twenty cycles of amplification were carried out (1 cycle:1 minute at 95°C, and three minutes at 72°C), after which products were combined, phenol extracted, ethanol precipitated, and resuspended in TE buffer, at a concentration of 0.5 µg/µl. Hereinafter, this is referred to as the representation.

### Example 4

The representation was then prepared for subtractive hybridization by removing SEQ ID NO: 1 therefrom by digestion with Dpn II. The resulting digest was phenol extracted and ethanol precipitated. In the case of the unstimulated sample, this resulted in the driver, while the stimulated sample resulted in the tester. Portions of tester (20 µg) were gel purified on a 1.2% agarose gel and isolated. Samples (2 µg), were ligated to SEQ ID NOS: 3 and 4, in the same way that SEQ ID NOS: 1 and 2 were ligated, as described, supra.

In a first cycle of subtractive hybridization, 0.4 µg samples of tester with SEQ ID NOS: 3 and 4 ligated thereto were mixed with 40 µg of driver cDNA. The mixture was phenol extracted, ethanol precipitated, dissolved in 2 µl of 3XEE buffer (30mM EPPS pH 8.0), 3mM EDTA; pH 8.0, 3 mM EDTA. This was overlaid with 30 µl of mineral oil, and denatured for five minutes at 98°C. A 5M NaCl solution (0.5 µl) was added, and DNA was hybridized for 20 hours, at 67°C. The reaction mixture was diluted to 200 µl with TE, and tRNA carrier. The samples were incubated for three minutes at 72°C to melt away SEQ ID NO: 4, and then four PCR reactions (200 µl) were prepared. These included 20 µl of diluted hybridization mix without primer, to fill in the ends of the reannealed tester, followed by 10 cycles of amplification after adding samples of SEQ ID NO: 3 (1 cycle:lminute at 95°C, three minutes at 70°C) after which products were combined, phenol extracted, ethanol precipitated, and resuspended in 40 µl of 0.2XTE buffer. Single stranded DNA was degraded by a 30 minute treatment of 20 µl of this material with 20U of mung bean nuclease, at a total volume of 40 µl. Samples was diluted (1:5), in 50 mM Tris-HCl, at pH 8.9, followed by five minutes of heating at 98°C to inactivate the enzyme. A second PCR was carried out, using 20 µl of the product described supra, 2 µl of SEQ ID NO: 3 (1 mg/ml), and 1 µl (5 U) of Taq DNA polymerase. A total of 18 cycles (1 cycle:1 minute at 95°C, three minutes at 70°C) were carried out. Products were combined, phenol extracted, ethanol precipitated, and resuspended at 0.5-1 µg/µl. The product is referred to hereafter as "DP1", or the first difference product.

### Example 5

DP1 was then digested with endonuclease DpnII, as described above, and was ligated to SEQ ID NOS: 5 and 6, following the same processes described for SEQ ID NOS: 1, 2, 3 and 4. Subtractive hybridization and selective amplification, as described in example 4, was repeated, and second difference product, or "DP2" , was generated. In these experiments, 50 ng of DP1 was the tester. The driver (40 µg), was as described supra. The process was repeated to generate a third difference product, using SEQ ID NOS: 3 and 4 as adapters. To generate the third product, 100 pg of tester were mixed with 40 µg of driver. All steps of the protocols supra were repeated, except the final amplification was carried out for 22 cycles, where one cycle was one minute at 95°C, and three minutes at 70°C. This yielded the final difference product.

### Example 6

The final difference products were digested with DpnII, and then cloned into the BamHI site of a commercially available vector, i.e., pTZ19R. Double stranded DNA plasmids were prepared, and then sequenced, using standard methods. The sequences were compared to known sequences in the GenBank and EMBL data bases, using a BLAST search program.

At the end of this subtraction procedure, a short cDNA fragment was identified, i.e., a fragment about 200 base pairs long. This fragment was used to screen a cDNA library from BW 5147 cells. The largest clone was sequenced. It is discussed infra. It does not correspond to any known sequence.

The nucelotide sequence (SEQ ID NO: 7), is 1119 bases long, including a 537 base pair open reading frame, which encodes a protein 179 amino acids long. The predicted molecular weight of the protein is 20,093. There are two additional ATG codons which, if they acted as start codons, would produce proteins 172 and 167 amino acids in length, with molecular weights of 19,335 and 18,770 daltons, respectively. Each form of the protein is characterized by a sequence of hydrophobic amino acids which would be cleaved off of the molecule via the endoplasmic reticulum to provide a mature protein.

Analysis of the sequence shows three AT rich motifs (TTATTTAT). These motifs are often found in 5'- untranslated regions of cytokines and oncogenes. Kruys, et al., Science 245: 852 (1989), have shown that these repeats modulate stability of mRNA.

### Example 7

The cDNA isolated and analyzed in example 6, supra, was then used as a probe to identify genomic DNA for TIFα.

A genomic library prepared from mouse strain 129 was screened with SEQ ID NO: 7, following standard methods. An EcoRI fragment from a positive clone was subcloned into plasmid pZERO and partially sequenced. The partial sequence is presented as SEQ ID NO: 8.

### Example 8

A second EcoRI fragment from the positive clone described in Example 7, supra, was also subcloned. There was a great deal of homology, but the sequences were not identical. To be specific, intron 1 of this sequence was 98 % identical to SEQ ID NO: 8, intron 2 was 100 % identical and intron 3 was 92 % identical.

What is striking about the sequences is that the promoters are not at all homologous, suggesting independent regulation. The 5' untranslated regions are 92 % identical. The first exon for TIFα is split into exon 1α and exon 1β. The first coding exon (which is exon 1b for TIFα and exon 1 for TIFβ) are 99.5 % identical, while the second exons are 100% identical, the third exons 97% identical, the fourth exons 98.5 % identical, and 96 % for the fifth exon. In the untranslated 3' - region, homology is 96%.

### Example 9

Using the information described in example 8, supra, a cDNA sequence for the second clone, designated TIFβ was deduced, and is set forth as SEQ ID NO: 9. The genomic DNA sequence was also ascertained, in the same manner as is described, supra, and is set forth as SEQ ID NO: 42. The amino acid sequence is SEQ ID NO: 41.

As compared to the coding region for TIFα, that of TIFβ has six silent changes. There are two changes which result in an inconsequential amino acid change (at both of positions 36 and 103, Val in TIFα becomes Ile in TIFβ). There is also a more significant change, at position 112, where Gln becomes Arg.

### Example 10

Experiments were undertaken to study expression of the TIFs. BW 5147 cells were stimulated with recombinant murine IL-9 (200U/ml), for varying periods of time (0.2, 0.5, 1, 2 & 24 hours). Total RNA was then isolated, using standard methods and reagents. Reverse transcription was then carried out, using 5µg total RNA and an oligo (dT) primer. Samples of cDNA corresponding to 20ng of total RNA were then amplified for 25 cycles using different primers. (One cycle was 4 minutes at 94 °C, 1 minute at 57°C, and 2 minutes at 72°C). The TIF primers were:
5'-CTGCCTGCTT CTCATTGCCC T-3' (SEQ ID NO: 10)
   and
5-CAAGTCTACC TCTGGTCTCA T-3' (SEQ ID NO: 11)
   (sense and antisense, respectively).

These correspond to nucleotides 107-127, and 766-786 of SEQ ID NO: 7, respectively. As a control, β-actin was amplified as well, for 18 cycles (first cycle: 4 minutes at 94°C, 1 minute at 60°C, 2 minutes at 72°C. Succeeding cycles were 1 minute at 94°C, 1 minute at 60°C, 2 minutes at 72°C).

Following amplification, post PCR products were analyzed on a 1 % agarose gel, and specific amplification was confirmed, following blotting, using internal radioactive probes. The probe for TIF was:
5'-GACGCAAGCA TTTCTCAGAG-3' (SEQ ID NO: 12)
the conditions and probes set forth were not specific for one or the other of the forms of TIF; however, the amplification product of TIFα contains a KpnI restriction site, while the restriction site for TIFβ does not. Digestion of the amplification products with KpnI indicated that most, if not all, of the TIF mRNA induced by IL-9 was TIFα, suggesting that the TIFα expression was induced rapidly via the IL-9. The mRNA for TIFα was detectable after 30 minutes of stimulation, and reached a plateau over a 1-24 hour time period.

### Example 11

Experiments were then carried out which showed that the induction of TIF mRNA by IL-9, described supra, does not require protein synthesis. In these experiments, total RNA was extracted from cells stimulated for 24 hours, as described in example 10, but with or without 10µg/ml of a protein synthesis inhibitor, cycloheximide, for 4.5 hours. In a parallel set of experiments, cells were not stimulated. The total RNA was extracted, and RT-PCR amplification was carried out as described in example 10. Post-PCR products were analyzed on an ethidium bromide-stained, 1 % agarose gel. What was seen was that the induction by IL-9 still occurred when protein synthesis was blocked. Hence, the effect of IL-9 is a direct effect, not requiring the synthesis of a protein mediator.

### Example 12

In these experiments, the role of STAT proteins in induction of TIF mRNA was studied on derivatives of the cell line BW5147. The first line, BWh9R, expresses wild type human IL-9 receptors. The line BW-Phe116 is a transfectant with a single mutation (at position 116), which renders the receptor unable to activate STAT transcription factors. Still another cell line, BW-mut6, has a mutation which renders the receptor unable to activate STATS, while retaining the ability to activate STAT1 and STAT3. Finally, cell line BW-mut7 has a single mutation which renders the IL-9 receptor unable to activate STAT1 and STAT3, but which retains the ability to activate STAT5.

Cell stimulation, isolation of total RNA, reverse transcription and amplification of cDNA were all carried out as described in example 10 (Cells were stimulated for 24 hours. Both human and murine recombinant IL-9 were used). The PCR products were analyzed on an ethidium bromide stained, 1% agarose gel, as describe supra.

The analysis revealed that human IL-9 did not induce expression in BW-Phe116, suggesting that STAT transcription factors are implicated. It was found that IL-9 induced TIF expression in the BW-mut6 mutant, but not the mut7 variant, suggesting that STAT1 or STAT3 are involved, but not STAT5.

### Example 13

The expression of TIF mRNA in normal mouse spleen cells was then studied. Spleen cells from 10-12 week old Balb/c mice were cultured for 24 hours in control medium or the control medium supplemented with 20µg/ml of LPS (which activates B lymphocytes and macrophages), or ConA (which activates T cells), or ConA plus 1 % of a blocking antiserum against murine IL-9, with β actin being used as a control. Purification of RNA, RT-PCR analysis were carried out as described supra.

The data indicated that TIF is, at best, very weakly expressed in resting spleen cells, not induced by LPS, but strongly induced by ConA. Anti IL-9 antiserum did not affect induction by ConA, suggesting that its effect is not mediated by IL-9, or is mediated by other cytokines.

When the ConA activated spleen cells were analyzed using sequences of RT-PCR products, it was found that these cells were expressing TIFα predominantly, or exclusively.

### Example 14

Further experiments showed that TIP mRNA was expressed even in the absence of IL-9 induction.

Spleen cells from 5 week old FVB mice were enriched for T cells, using a nylon wool column. Then, the cells were stimulated for 24 hours in medium supplemented with ConA (a T cell activator), or PMA (which activates PKC in most cells), either with or without IL-9.

Total RNA was isolated using standard techniques, and then ten microgram samples were fractionated via electrophoresis on a 1.3 % agarose gel containing 2.2M formaldehyde. The fractions were then transferred to a nitrocellulose membrane, labeled, and assayed in a hybridization assay following Van Snick, et al, J. Exp. Med. 169: 363 (1989).

The results indicated that the induction of TIF by ConA was not modified, and that IL-9 did not induce TIF RNA in PMA activated spleen cells.

### Example 15

The expression of TIF mRNA in various cell lines was tested. In these experiments, murine cell lines were stimulated for at least one day, with a particular cytokine. Specifically, 9T7 is a T cell lymphoma, which responds to IL-2, IL-4 or IL-9. Cell lines TS3 and TS6 are derived from T helper cell clones, and proliferate in the presence of either IL-2 or IL-9. MC9 and LI38 are mast cell lines, which proliferate in the presence of either IL-3 or IL-9.

Following stimulation, total RNA was prepared using standard guanidium isothiocyanate lyses, and CsCl gradient centrifugation.

The 9T7 line was then analyzed by Northern blotting, as described in example 14, while the other lines were assayed using RT-PCR analysis, as described supra.

It was found that IL-9 upregulated TIF expression in T helper cells and mast cells, while IL-2 and IL-3 did not. The 9T7 cell line, however, showed roughly the same level of expression, regardless of the cytokine, indicating that IL-9 is not mandatory for TIF expression.

### Example 16

The expression of TIF mRNA in B cell lines was then studied. The cell lines A20, 70Z/3, and BCL-1 are B cell leukemia cell lines which grow, in vitro, without cytokines. These cells were stimulated for 24 hours with IL-4 and IL-9 and total RNA was isolated, using standard methods. Expression was analyzed by RT-PCR which was carried out for 35 cycles, followed by blotting and hybridization, as described supra.

The results indicated that TIF expression is detectable in B cells, but is weakly upregulated at best in the presence of IL-9 and IL-4.

### Example 17

Experiments were then carried out to study expression of the inventive molecules in T helper cell lines. TS2 and TS1 are known T helper cell lines, derived from T helper cell clones, which proliferate in the presence of either IL-9 or IL-2 (TS2), and either IL-9 or IL-4 (TS1). Specifically, TS1 or TS2 cells were grown in the presence of the listed cytokines for at least 10 days, after which RNA was extracted using known methods. Expression of the molecules was studied via RT-PCR (35 cycles), using the protocols described supra. In TS1 cells both IL-4 and IL-9 induce TIF expression, but IL-2 does not do so in TS2 cells.

### Example 18

Expression of TIF mRNA in various mouse organs were studied. Total RNA was prepared from liver, kidney, heart, brain, intestine, spleen, thymus, lung, muscle and bone marrow, using standard guanidium isothiocyanate methodologies and CsCl gradient centrifugation. Forty cycles of RT-PCR were carried out, using the protocols described supra. Strongest expression was found in thymus tissue, while less intense signals were found in brain tissue, and weaker expression in the remaining tissues.

### Example 19

The following experiments describe production of TIFα in 293-EBNA cells.

Complementary DNA for TIFα was described supra. It was subcloned into a commercially available expression vector pCEP-4, in operable linkage with a CMV promoter. The resulting plasmids were transfected into 293-EBNA cells, using standard lipofectamine methods. Following transfection, the cells were incubated in a methionine free medium, supplemented with ³⁵S labeled methionine, for 24 hours. Supernatant was harvested, and run on an acrylamide gel, followed by electrophoresis. The gel was then dried and exposed to autoradiography for 1 day. A control was then run by transfecting cells with the same plasmid, in which the cDNA was cloned in the antisense direction.

A heterogenous band of about 25-30 kilodaltons was found from the cells transfected with TIF in the sense direction. Any discrepancies between the predicted molecular weight, the actual molecular weight in the system, and the heterogeneity, can be attributed to glycosylation. In a series of parallel experiments, cDNA encoding human TIF was expressed in the same way as the murine cDNA was expressed. With the exception of the change of the cDNA, all experimental parameters were the same.

### Example 20

Further experiments were carried out to study production of TIFα in COS cells. Specifically, TIFα cDNA was subcloned into the plasmid pEF-BOS.puro described by Demoulin et al., supra, in operable linkage with the EF-1α promoter. The plasmid cDNA was transfected into COS cells, using the same lipofectamine method described supra. The cells were incubated in methionine free medium, supplemented with ³⁵S methionine for 24 hours, after which supernatant was treated as described in example 19, supra. Again, a heterogenous band of 25-30 kilodaltons was observed, as well as an 18 kilodalton band, which probably represents a non-glycosylated form of the molecule.

### Example 21

In these experiments, it was discovered that TIF induces STAT activation in mesangial, neuronal melanoma, and hepatoma cells. It is known that when cytokines activate STAT factors, the factors dimerize, move from cytoplasm to the nucleus, and bind to target sequences in promoters. The details of the experiments follow.

Transfected 293-EBNA cells as described supra were used following incubation in normal medium for 48 hours, as were supernatant from the controls, also described supra. Samples of a mouse kidney mesangial cell line, ("MES13" hereafter), a rat pheochromocytoma cell line, ("PC12" hereafter), four different human melanomas (SK23, AUMA, NA-8mel and MULL), human hepatoma (HepG3) and rat hepatoma (H-4-II-K) were used. Cell samples (0.5x10⁶) were stimulated for 5-10 minutes in the presence of 1% of supernatant. Nuclear extracts were then prepared, in accordance with Demoulin et al., Mol. Cell. Biol. 16: 4710 (1996). In brief, cells were washed with PBS and then resuspended in 1 ml of ice cold hypotonic buffer for 15 minutes. (Buffer was 10mM HEPES buffer, pH 7.5, with 10mM KCl, 1mM MgCl₂, 5% glycerol, 0.5 mM EDTA, 0.1mM EGTA, 0.5mM dithiothreitol, and 1mM Pefabloc, 1mM Na₃V₄, and 5mM NaF). Cells were then lysed by adding 65 µl of NP-40, followed by vortexing. Nuclei were pelleted, by vortexing for 30 seconds at 14,000 rpm, followed by extraction in buffer supplemented with HEPES (20mM), glycerol (20%), and NaCl (420mM). Nuclear debris was removed by centrifuging for 2 minutes. DNA binding activity was determined in accordance with Demoulin et al., supra, using a ³²P labeled double stranded oligonucleotide called "GRR," which contains the STAT binding site of the FcγRI gene promoter, i.e.:
5'ATGTATTTCC CAGAAA-3' (SEQ ID NO: 13)
   and
5'-CCTTTTCTGG GAAATAC-3' (SEQ ID NO: 14)
corresponding to the upper and lower strands of the binding sites in the GRR probe. Briefly, 5µl volume of nuclear extracts were incubated in binding buffer (12mM HEPES, pH 7.6, 10mM KCl, 0.5mM EDTA, 2.5% glycerol, 0.1mg of poly(dI-dC) per ml) for 5 minutes. Radiolabeled GRR probe (10⁵cpm; approximately 0.5ng) was added, and incubation was continued for 25 minutes before loading onto a non-denaturing polyacrylamide gel.

It was also noted that the complexes observed in MES13 cells, described supra, were partially overshifted by both anti-STAT5 and anti-STAT3 antibodies, showing that (i) the cells under examination were targets for TIF, and (ii) that STAT3 and STAT5 are major components of the complex activated by TIF. The difference in STAT profile, as compared to the profile in Example 12, supra, is attributable to the difference in cell source (human versus mouse). It was also observed that human TIF works on murine cells, and vice versa.

### Example 22

This example details the isolation and cloning of a nucleic acid molecule which encodes human TIF. First, human peripheral blood mononuclear cells were prepared via standard density gradient centrifugation. Following this preparation, samples were cultured for 24 hours, at 3 x 10⁶ cells/ml, either with or without anti-CD3 monoclonal antibody (The antibody was the commercially available OKT3 mAb, used in the form of ascites fluid at 1/500 dilution). This antibody was used because T cell derived cytokines are generally expressed only upon activation by e.g., CD3 specific antibodies.

Total RNA was isolated from these cells, using standard guanidine-isothiocyanate / CsCl ultra-centrifugation techniques. Following isolation, 10µg samples of the RNA were reverse transcribed using an oligo (dT) 15 primer.

Following preparation of cDNA, as outlined supra, samples which corresponded to 100ng of total RNA were amplified, via PCR, using the following primers:
5' - AGGTGCTGAA CTTCACCCTG GA - 3' (SEQ ID NO: 15)
5' - CCACTCTCTC CAAGCTTTTT CA - 3' (SEQ ID NO: 16)
which are based upon a murine cDNA sequence, (i.e., SEQ ID NO: 7). The PCR conditions involved 30 cycles of amplification, with one cycle defined as 1 minute at 94°C, followed by 1 minute at 42°C, and then 2 minutes at 72°C. Amplification product was separated on an agarose gel, using standard methods, and then sequenced. The result indicated that fragments of the cDNA had been amplified. Hence, a second reaction was carried but, using the same materials except SEQ ID NO: 16 was replaced by SEQ ID NO: 17, i.e.:
5' -CAAGTCTACC TCTGGTCTCA T- 3'
This second PCR reaction was carried out for 25 cycles, with one cycle being defined as 1 minute at 94°C, followed by 1 minute at 45°C, and then 2 minutes at 72°C. The amplification product was subjected to the same steps as the first one. A fragment of 418 nucleotides was amplified, which was found in anti-CD3 stimulated cells, but not resting PBMCs. This fragment's nucleotides sequences is set forth at SEQ. ID NO: 18.

### Example 23

Following preparation of amplification product, the 5' end of cDNA was isolated by using standard, 5' - RACE techniques. In brief, first strand cDNA was prepared by using SEQ ID NO: 19 as a primer, i.e.:

5' - TGGCCAGGAA GGGCACCACC T - 3' This primer was based upon the sequence information obtained in accordance with example 22. In brief, the 5' - RACE method was carried out by combining 1 µg of total RNA, prepared as described supra, 2.5 pmoles of SEQ ID NO: 19, reverse transcriptase , reverse transcriptase buffer, 2.5 µl of dNTP mix (10 mM), 2.5 µl of MgCl₂ (25mM), and 2.5 µl of dithiothreitol (0.1 M). The reaction was carried out and, after completion, original RNA was removed via adding RnaseH, and Rnase TI. Any unincorporated dNTPs, as well as primer and proteins, were removed. The cDNA was tailed using terminal transferase, or "TdT." This enzyme creates a 3'-binding site for the abridged anchor primer, as described infra. Tailing was carried out by combining the purified, first strand cDNA, TdT, buffer (10 mM Tris-HCl, 25 mM KCl, 1.5 mM MgCl₂), and 200 µM of dCTP.

Following the tailing reaction, PCR was carried out using:
5' -CCTATCAGAT TGAGGGAACA G - 3' (SEQ ID NO: 20)
   and 5' - RACE abridged anchor primer:
5' - GGCCACGCGT CGACTAGTAC GGGIIGGGIIGGGIIG - 3' (SEQ ID NO: 21). The amplification involved 35 cycles (1 cycle defined as 1 minute at 94°C, 1 minute at 56°C, and 2 minutes at 72°C). Following this, nested amplification was performed on 5 µl of a 1/100 dilution of the amplification product, using SEQ ID NO: 20 and the abridged universal amplification primer:
5' - GGCCACGCGT CGACTAGTAC - 3' (SEQ ID NO: 22).
Amplification involved 30 cycles (1 cycle being defined as 1 minute at 94°C, 1 minute at 56°C, and 2 minutes at 72°C). The resulting PCR product was cloned, following standard procedures, and sequenced.

These three protocols, i.e., the two experiments described supra which generated fragments, and the 5' - RACE PCR, also described supra, permitted alignment of the sequenced amplification product, to generate the complete sequence.

Following the alignment, oligonucleotides were generated which flanked the deduced open reading frame, i.e.:
5' - CCTTCCCCAG TCACCAGTTG - 3' (SEQ ID NO: 23)
   and
5' - TAATTGTTAT TCTTAGCAGG - 3' (SEQ ID NO: 24).
These primers were used to amplify the entire open reading frame, using mRNA from CD3 specific mAb stimulated cells, as described supra. For amplification, 25 cycles (1 cycle being defined as 1 minute at 94°C, 1 minute at 56°C, and 2 minutes at 72°C).

The complete sequence of the human cDNA is set forth at SEQ ID NO: 25. It contains a 537 base pair ORF which encodes a 179 amino acid protein. This is the same length as the murine protein. The human protein has 79 % amino acid homology with the murine protein, and 25% homology with IL-10. Human and murine proteins are set out as SEQ ID NOS:43 and 40, respectively.

### Example 24

These experiments detail work on the isolation of human genomic DNA corresponding to the cDNA discussed supra.

Based upon the cDNA sequences, primers were developed which correspond to nucleotides 51-70 and the complement of nucleotides 631-650 of SEQ ID NO: 25. PCR was carried out, using standard methodologies. Specifically, 100ng of genomic DNA taken from CESS cells (an EBV- transformed, lymphoblastoid cell line) was used as a template, and 33 cycles of amplification were carried out (one cycle of amplification being defined as 94° C for 30 seconds, 50° C for 30 seconds, and 72° C for 5 minutes). Once a sequence was isolated, it was sequenced, and this is set forth as SEQ ID NO: 26. The sequence is about 4.8 kilobases in length, and is believed to contain the entire genomic sequence encoding the TIF molecule, lacking only the 5' flanking region, the promoter, and the 3' end. Analysis indicates that it contains 6 exons and 5 introns, as does the murine genomic sequence.

Southern blot hybridization was carried out, using standard methods. It showed that the genome contains only a single copy of the TIF gene.

### Example 25

It was of interest to identify where the genomic DNA discussed supra was located in the human genome. In order to do this, two different approaches were taken. In the first, the sequence discussed supra, i.e., SEQ ID NO: 26, was labeled with a flourescent label, and then was used to probe the human genome via fluorescent, in situ hybridization ("FISH") using standard methods.

In a second approach, a panel of radioactive hybrid clones were screened using the probe consisting of nucleotides 51-70 of SEQ ID NO: 25, and 5'-ATCAGATGGA TTACTGAATG-3' (SEQ ID NO:27). PCR was carried out using 25 ng of genomic DNA as a template, for 35 cycles, where one cycle is defined as 94 °C for 1 minute, 55° C for 1 minute and 72 °C for 2 minutes.

Both methodologies indicated that the gene is located at chromosome 12q15. Some work links diseases associated with asthma at this site. See, e.g. Nat. Genet. 15:389-392 (1997); Ober, et al, Hum. Mol Genet. 7(9): 1393-1398(1998); Nickel, et al, Genomic 46(1):159-162(1997); Takahashi, et al, Genomics 44(1):150-2(1997); Barnes, et al, Genomics 37(1):41-50(1996).

Public databases were consulted to determine if any part of the sequences were presented therein. The last exon of TIF was found on a BAC clone, derived from chromosome 12q15. (Accession No: AC007458; 191,111 base pairs, BAC RDCI11-444B24). The identification of the last exon on this clone suggests that the TIF gene is located about 90 kilobases from the IFN gene, and less than 30 kilobases from a gene referred to as AK155, which is an IL-10 related cytokine (Knappe, et al, J. Virol 74:3881-3887 (2000)).

### Example 26

These experiments describe the manufacture of antibodies which bind to the TIF protein. To make these, a peptide consisting of amino acids 40-61 encoded by SEQ ID NO: 7 was coupled to KLH carrier protein, using standard methods and a ratio of 1 mg peptide to 1 mg carrier protein. Subject animals (rabbits), were immunized 3 times, at 2 week intervals, with 150 µg of the complex. The immunogen was emulsified in Complete Freund's Adjuvant for the first injection, and then Incomplete Freund's Adjuvant for the next two.

A first bleed was performed one month after the last injection, and serum was prepared, following known methods.

The serum was then tested in a standard Western Blot. In brief, 10 µl of supernatant from cells transfected with either SEQ ID NO: 7 or SEQ ID NO:25 were separated via SDS-PAGE electrophoresis, and then blotted onto PVDF membranes. Antiserum was diluted to 1:500, and used in a standard Western Blot protocol, together with anti-rabbit antibody as the secondary antibody, and a commercially available detection kit.

It was found that the serum did, in fact, recognize the TIF protein.

### Example 27

This example describes experiments carried out to identify cell lines which are responsive to human TIF. HEK293-EBNA human embryonic kidney cells, described supra, were seeded in 6 well plates, at 3 x 10⁵ cells/well one day before transfection. They were transfected with 2 µg of pCEP-4 plasmid which contained cDNA for human TIF, under control of the CMV promoter. Cells were incubated after transfection, in 1.5 ml normal medium, for 3 days, in order to maximize production of recombinant human TIF.

It was hypothesized that human TIF would induce activation of STAT transcription factors in the same way the murine factor did. The protocol of example 21, supra was followed. Supershifts were performed by adding anti-STAT antibodies (0.75µg anti STAT 1, 1 µg of anti-STAT 3, or 1 µg of anti-STAT 5b), to mixtures of nuclear extracts and labeled DNA probe, and incubated.

A TIF induced bandshift was observed when the hepatoma cell line HepG2 was used. The antibodies described supra were used to characterize the response further. Anti-STAT-3 antibodies shifted most of the retardation complexes, while the weak, remaining complexes were supershifted by anti-STAT-1 antibodies. The anti-STAT-5 antibodies had no effect. This indicates that STAT-3 and, to a lesser extent, STAT-1 are the major transcription factors activated by TIF. These results were also obtained using human hepatoma cell line HepG3, and also hepatoma cell line H4IIE.

### EXAMPLE 28

This example describes experiments designed to measure STAT activation by means of a reporter gene. The reporter was "pGRR5." This contains 5 copies of the sequence set out in example 21, upstream of a luciferase gene under control of the TK promoter. The control was vector pRL-TK, which contains the *remilla* luciferase gene, under control of the TK promoter.

The assay was carried out by combining 10⁶ HepG2 cells with 15 µg pGRR5, and 1 µg of Prl-TK (250V, 74Ω, 1,200 µF). The pool of transfectants was divided into 24-well plates (42,000 cells/well).

After 1 hour, cells were stimulated with either human TIF (1% HEK293 cell supernatant) with 300U/ml of human IL-6, 1% supernatant from mock transfected HEK293 cells, or medium alone.

After two hours, cells were pelleted, and lysed. Luciferase activity was monitored using standard methodologies. The results indicated that stimulation with the molecule of the invention increases transcriptional activity of a promoter that includes STAT-binding sites.

### EXAMPLE 29

Activation of STAT-3 by cytokines like IL-6 is known to result in acute phase protein induction in hepatoma cells. To determine if TIP exerted the same activity, 5x10⁶ HepG2 cells were stimulated for 2, 13, or 24 hours, with 1% supernatant from transiently infected HEK293-EBNA cells. In some experiments protein synthesis inhibitor cycloheximide was used at 10 µg/ml, and combined with the cells and supernatants. Following stimulation, total RNA was isolated using standard methodologies, and reverse transcription was performed on 10 µg samples of total RNA, using an oligo(dT) primer. Then, cDNA corresponding to 20 ng of RNA was amplified, for 18 cycles, with primers specific for human serum amyloid A ("SAA"), i.e.:
agctcagcta cagcacagat
   (sense, SEQ ID NO: 28)
cctgccccat ttattggcag
   (antisense, SEQ ID NO: 29)
Human α1 antichymotrypsin:
tgtcctctgc caccctaaca
   (sense, SEQ ID NO: 30)
taattcacca ggaccatcat
   (antisense, SEQ ID NO: 31)
   for human haptoglobin:
gtggactcag gcaatgatgt
   (sense, SEQ ID NO: 32)
acatagagtgt taaagtggg
   (antisense, SEQ ID NO: 33)
   and for human β-actin:
gctggaaggt ggacagcgag
   (sense, SEQ ID NO: 34)
tggcatcgtg atggactccg
   (antisense, SEQ ID NO: 35).
For SAA, Tm was 54°C, while it was 52°C for human α1 - antichymotrypsin and haptoglobin, and 56°C for β-actin. PCR products were analyzed, in ethidium bromide stained agarose gels, using standard methods.

The results indicated that TIF strongly induced SAA and α1-chymotrypsin and, to a lesser extent, haptoglobin. In order to determine if TIF directly upregulates SAA, or whether protein synthesis is required, HEPG2 cells were stimulated, as described, in the presence of cycloheximide. SAA expression was not affected, indicating that protein synthesis was not required for TIF activity.

### EXAMPLE 30

The results, supra, show that TIF and IL-6 appear to have similar activities on acute phase reactants. As IL-6 activity is mediated through gp 130, experiments were carried out to determine if TIF activity is mediated through gp 130 as well.

To determine if this was the case, HepG2 cells were transfected with a luciferase reporter, as described, supra, and were then stimulated with either TIF or IL-6, in the presence of polyclonal, anti-gp 130 antibodies which had been determined previously to block the activity of gp 130 interacting cytokines.

The results indicated that the polyclonals blocked only IL-6 activity.

Parallel experiments were carried out to determine if the IL-10R β chain was involved. In the presence of anti - IL-10R β antibodies, TIF activity was blocked completely while IL-6 activity was unaffected. The same affect was observed upon assaying for SAA expression.

### EXAMPLE 31

These experiments were designed to determine the ability of TIF to regulate acute phase proteins in vivo.

Varying amounts of recombinant murine TIF (50, 12.5, 3.2, 0.8 or 0.2 µg) were injected, intraperitoneally, into endotoxin resistant C3H/HeJ female mice (10-12 weeks old). Six hours after injection, the mice were killed, with the exception of the mice who received 50 µg of TIF, which were killed after 1, 3, 6, 12, or 24 hours. Livers were removed and directly frozen in liquid nitrogen. Total RNA was then extracted, using standard methodologies, after which 10 µg samples of total RNA were fractionated on a 1.3% agarose gel which contained 2.2 ml/liter formaldehyde. Samples were then transferred to nitrocellulose membranes.

Murine SAA probes were manufactured, using a commercial labelling kit. The hybridization assay was carried out in accordance with Van Snick, et al, J. Exp. Med 169:363-368 (1989). The probe itself was obtained via PCR, as described supra, using cDNA from murine liver, isolated as described supra. The primers used to manufacture the probe were:
tctgctccct gctcctggga
   (sense, SEQ ID NO: 36)
   and
tccaggaggt ctgtagtaat
   (antisense, SEQ ID NO: 37)
The results indicated that the highest dose of the murine TIF (50 µg), induced SAA expression in as little as 1 hour post i.p. injection. Maximal effect was reached after 6 hours. The expression level for SAA decreased at 24 hours past injection.

The data generated from the experiments using varying doses of TIF indicated that maximal induction of SAA still resulted when 3.2 µg dosages were used. SAA message was still detectable when as little as 0.8 µg were used.

### EXAMPLE 32

Initially, TIF was identified as a T cell derived cytokine. Most cytokines that regulate the liver acute phase are produced mainly during inflammation. In order to determine if TIF could be produced via inflammatory stimulation, in vivo, 2 µg of E.coli lipopolysaccharide antigen were injected, intraperitoneally into 12 week old, female BALB/c mice. Mice were killed two hours later, and organs were frozen in liquid nitrogen. Total RNA was isolated following standard protocols, and reverse transcription was performed on 10 µg total RNA, using oligo (dT) primers. Following the reverse transcription, cDNA corresponding to 20 ng of total RNA was amplified for 25 cycles, using primers specific for TIF, i.e.:
ctgcctgctt ctcattgccc t
   (sense, SEQ ID NO: 38)
   and
caagtctacc tctggtctca t
   (antisense, SEQ ID NO: 39),
which had a Tm of 55°C. PCR products were analyzed via agarose gel electrophoresis.

The results indicated that LPS induced expression of TIF in all organs examined, indicating that TIF is involved in inflammatory processes.

The foregoing examples describe the invention. Described herein are isolated nucleic acid molecules, which encode TIF proteins such as those with the amino acid sequence of the protein encoded by the nucleotide sequence of SEQ ID NO: 7, 25 or 26. It will be appreciated by one of ordinary skill that the degeneracy of the genetic code facilitates the preparation of nucleic acid molecules which may not be identical to the nucleotide sequence of SEQ ID NO: 7, 25 or 26, but which encode the same protein. Genomic DNA, complementary DNA, and RNA, such as messenger RNA, are also described, as well as isolated nucleic acid molecules from other animal species, including other mammals. Also described are isolated nucleic acid molecules whose complements hybridize to SEQ ID NO: 7, SEQ ID NO: 8, SEQ ID NO: 9, or SEQ ID NO: 25 or SEQ ID NO: 26 under stringent conditions. "Stringent conditions," as used herein, refer, for example, to hybridization at 65°C in buffer (3.5xSSC), 0.02% Ficoll, 0.02 % polyvinylpyrrolidone, 0.02% bovine serum albumin, 25mM NaH₂PO₄ (pH 7), 0.1% SDS, 2mM EDTA, followed by a final wash at 2xSSC, room temperature and then 0.1xSSC/0.2xSDS at temperatures as high as, e.g., about 65°C. More stringent conditions, such as 0.1xSSC, can also be used. These nucleic acid molecules encode proteins of about 17-22 kD as determined by SDS - PAGE, which activates STAT proteins, such as STAT 1, STAT3 and/or STAT5. In glycosylated form, these proteins can range from about 17 to about 30 kilodaltons, as determined by SDS - PAGE. Also described are isolated nucleic acid molecules which encode proteins having at least 30%, preferably at least 45%, more preferably at least 60%, and most preferably 90 % amino acid identity with an amino acid sequence of a protein encoded by SEQ ID No: 7, 25 or 26, e.g., SEQ ID NO: 42.

Also described are expression vectors which include the nucleic acid molecules herein, operably linked to a promoter, so as to facilitate expression of the DNA. It is well within the skill of the artisan to prepare such vectors.

The vectors, as well as the nucleic acid molecules per se, can be used to prepare recombinant cells, be these eukaryotic or proharyotic, wherein either an expression vector or the nucleic acid molecule itself is incorporated therein, such as E. coli cells, COS cells, CHO cells, etc..

Proteins encoded by the above referenced nucleic acid molecules, preferably in isolated form, are also described. By "protein" is meant both the immediate product of expression of the nucleic acid molecules, glycosylated forms of it, as well as multimeric forms, such as dimers, trimers, and so forth. Also described are multimers, such as dimers, which contain at least one protein molecule of the invention, and at least one, different protein molecule. Preferably, this different protein molecule is a cytokine, such as IL-10. Also described are constructs, such as fusion proteins, where all or a part of the proteins described supra are linked in some fashion, such as in a fusion protein, to at least one additional protein or peptide, or amino acid sequence. The "fusion partner" may be, for example, a molecule which provides a recognizable signal, either directly or indirectly, such as a FLAG peptide, β-galactosidase, luciferase, and so forth. These fusion partners are preferably joined to the molecule which is described supra at the N- and/or C- terminus of the protein; however, it is to be understood that there are many techniques known for joining molecules to amino acids, and any and all of these methodologies can be used.

The individual protein molecules herein, as noted supra, will preferably have a molecular weight of from about 17 to about 30 kilodaltons, as determined by SDS-PAGE. In multimeric forms, the molecular weight of the complex will, of course, vary, but the TIF molecules contained therein will each have a molecular weight of about 17 to 30 kilodaltons, as determined by SDS-PAGE.

The proteins preferably consist of at least about 120 and no more than about 200 amino acids. Preferably, the amino acids sequences consists of or comprises all or part of the amino acid sequences encoded by SEQ ID NOS: 7, 8, 9, 25 or 26. More preferably, the amino acid sequence contains all but about the first 40 amino acids encoded by said SEQ ID's. Even more preferably, it contains all but about the first 20 amino acids encoded by these sequences. Most preferably, the protein comprises amino acids set forth at SEQ ID NO: 40 or 41, as well as proteins defined by the amino acid identities set out supra.

It will be appreciated by the skilled artisan that the proteins encoded by the above recited nucleic acid molecules may be used to produce antibodies, in accordance with standard protocols, in monoclonal and polyclonal form, as well as do fragments of said antibodies, chimeric forms, humanized forms, recombinant forms, and so forth. Also described are immunogens, comprising all or a part of the amino acid sequence protein molecules of the invention, preferably combined with an adjuvant, such as Complete or Incomplete Freund's Adjuvant. Portions of the protein sequences may be linked to other molecules, such as keyhole limpet hemocyanin, to render them more immunogenic. These antibodies can be used, e.g., to determine if the proteins are present. It has been shown, in the examples, that the nucleic acid molecules herein were expressed in the presence of the IL-9. Hence, a further feature described is a method to determine if IL-9 is or has been present, wherein one detects either the proteins of the invention, using antibodies for example, or mRNA using the nucleic acid molecules of the invention, as probes. The mRNA can be determined directly, or in the form of cDNA. Such probes may or may not be labeled, as a matter of choice for the user. Hence, one can determine, for example, if, following administration of IL-9, the cytokine is still efficacious, by determining if the nucleic acid molecule of the invention is present. This type of assay can be adapted, for quantitative studies, wherein one determines, for example, either if a cell is sensitive to IL-9, and if so, how sensitive it is. One can also use the proteins of the invention to phosphorylate STAT proteins such as STAT1, STAT3 and/or STAT 5. This in turn results in dimerization of the STAT protein, followed by migration to the nucleus to provoke the effect that these STAT proteins have on cells.

One could also use these molecules to test the efficacy of EL-9 agonists or antagonists when administered to a subject, such as a subject suffering from lymphoma, an immune system disorder such as an allergy, acquired immune deficiency syndrome, autoimmune diabetes, thyroiditis, or any of the other conditions described in, e.g, U.S. Patent No. 5,830,454; 5,824,551, and 5,935,929. The molecules can also be used to mediate the role of IL-9 in these and other conditions. To elaborate, since IL-9 induces TIFs, the TIFs are useful as IL-9 activity mediators. Thus, also described is a method to determine activity of endogenous IL-9, such as in situations where excess IL-9 activity is implicated, such as asthmas, allergies, and lymphomas. One can also block or inhibit IL-9 activity by blocking or inhibiting TIF or TIF activity, using, e.g., antisense molecules, antibodies which bind to TIF, or other antagonists of these molecules, such as, muteins of TIF, which bind to the TIF receptor but do not activate it, thereby inhibiting IL-9 induced activity.

Examples of conditions which can be treated by the use of such TIF muteins are allergies, asthma, and so forth. Muteins can be made in accordance with, e.g., Weigel, et al, Eur. J. Biochem 180(2):295-300(1989) and Epps, et al, Cytokine 9(3):149-156(1997). Such muteins can be used in the treatment of asthma, allergies, or both. Further, it will be clear to the skilled artisan that the models set forth, supra, can also be used to screen for appropriate muteins. The ability to regulate IL-9 activity is important in conditions such as those listed supra, as well as conditions such as apoptosis, including cortisol induced apoptosis, conditions involving the nuclear expression of BCL-3, since IL-9 is known to induce such expression, and so forth. "Antibodies," as used herein, refers to any portion of an antibody which binds to TIF, including chimeric and humanized antibodies.

Another feature described relates to the ability of the TIF type molecules to either promote regeneration or inhibit differentiation of tissue types on which the molecules are active. As was shown, supra, the TIF molecules target various cancer and normal cell lines (i.e., mesangial and neuronal cells, as well as melanoma and hepatoma cells). Hence, one can stimulate regeneration of tissue via, e.g., adding an amount of a TIF type molecule to a sample in need of regeneration of a tissue acted on by the TIF molecule. This approach can be used both in vitro, and in vivo. Similarly, antagonists of TIF may be added when the situation is one where the aim is to inhibit differentiation of a particular type of tissue, such as melanoma or hepatoma.

The genes which encode TIF, as noted in Example 25, supra, are located on chromosome 12. This chromosome is associated with asthma, as is known in the art. It is also known that region 12q15 is associated with other inflammatory disease. Also described is a method for determining susceptibility to conditions such as, or related to asthma, by determining if aberrations, such as polymorphisms, deletions, additions, etc., are present at the site of the TIF gene. Such aberrations may be an indicia of susceptibility to, or of the presence of, asthma, an allergic condition, or one or more related conditions. The ability to detect aberrations in a DNA sequence is well known in the art, and such methods need not be set forth herein. Preferably, the aberration or aberrations is detected via standard techniques, such as PCR, using the methodologies and primers referred to supra.

The data set forth supra, in particular the induction of expression of the molecule of the invention by LPS, and the regulation of the acute phase response by this molecule, indicate that the molecules herein are actively involved in the inflammatory response. Also described is the identification of proinflammatory and anti-inflammatory agents via use of IL-TIF/IL-21. It will be clear to the skilled artisan that IL-TIF/ IL-21 can regulate the inflammatory response. An aspect of this regulation is the modulation of the acute phase response by organs, such as the liver, by administering either IL-TIF/IL-21, or an antagonist thereof. See, e.g., Janeway, et al., Immunobiology (4th edition). Janeway explains that various cytokines such as IL-1, IL-6 and TNF-α activate hepatocytes to synthesize acute phase proteins, such as c-reactive protein, and mannan binding lectin, as well as those described in the examples, supra.

The role of IL-TIF/EL-21 in activating acute phase proteins also leads to a method for identifying agonists and antagonists of IL-TIF/IL-21, by determining if acute phase protein production changes in the presence of the putative agonist or antagonist, and IL-TIF/IL-21. An increase in production should be taken as an indicia that the test molecule is an agonist, and vice versa.

The invention concerns methods for regulating activity of IL-TIF/IL-21 in view of its relationships to interleukin-10 receptors. As was shown, supra, the use of IL-10Rβ antagonists, such as antibodies, inhibit IL-TIF/IL-21 activity. Hence, the invention is the use of agonists and antagonists of IL-10 receptors, such as IL-10Rβ agonists, and antagonists, to regulate IL-TIF/IL-21 production.

Other features of the invention will be clear to the artisan and need not be discussed further.
## Claims

1. An *in vitro* method for the inhibition of STAT-1, STAT-3 and/or STAT-5 activation by IL-TIF/IL-21, which comprises addition to a sample comprising IL-TIF/IL-21 and one or more of STAT-1, STAT-3 and STAT-5 of an antibody to IL-10Rβ, wherein IL-TIF/IL-21 is selected from:
(a) a polypeptide encoded by a sequence selected from SEQ ID NO: 7, SEQ ID NO: 25 or SEQ ID NO: 26; and
(b) a polypeptide having at least 60% amino acid identity to a polypeptide of (a).

2. The method of claim 1 wherein said antibody binds specifically to an IL-10Rβ molecule.

## Patentansprüche

1. In-vitro-Verfahren zur Inhibierung der STAT-1-, STAT-3- und/oder STAT-5-Aktivierung durch IL-TIF/IL-21, das die Zugabe zu einer Probe umfasst, die IL-TIF/IL-21 und ein oder mehrere STAT-1, STAT-3 und STAT-5 eines Antikörpers gegen IL-10Rβ umfasst, worin IL-TIF/IL-21 aus Folgendem ausgewählt ist:
(a) einem Polypeptid, das von einer Sequenz kodiert wird, die aus Seq.-ID Nr. 7, Seq.-ID Nr. 25 oder Seq.-ID Nr. 26 ausgewählt ist, und
(b) einem Polypeptid, das zumindest 60 % Aminosäure-Identität mit einem Polypeptid aus (a) aufweist.

2. Verfahren nach Anspruch 1, worin der Antikörper spezifisch an ein IL-10Rβ-Molekül bindet.

## Revendications

1. Procédé in vitro pour l'inhibition de l'activation de STAT-1, STAT-3 et/ou STAT-5 par l'IL-TIF/IL-21, qui comprend l'addition à un échantillon comprenant de l'IL-TIF/IL-21 et un ou plusieurs de STAT-1, STAT-3 et STATS-5 d'un anticorps dirigé contre l'IL-10Rβ, dans lequel l'IL-TIF/IL-21 est sélectionnée parmi :
(a) un polypeptide codé par une séquence choisie parmi SEQ ID N° 7, SEQ ID N° 25 ou SEQ ID N° 26 ; et
(b) un polypeptide ayant au moins 60 % d'identité d'acides aminés avec un polypeptide de (a).

2. Procédé selon la revendication 1 dans lequel ledit anticorps se lie spécifiquement à une molécule d'IL-10Rβ.


<110> Dumoutier, Laure Renauld, Jean-Christophe
<120> Isolated Nucleic Acid Molecules which Encode T Cell Inducible Factors, or Interleukin-21, The Proteins Encoded, and Uses Thereof
<130> LUD 5664
<140>
   <141>
<150> US09/419.568
   <151> 1999-10-18
<150> US09/354,243
   <151> 1999-07-16
<150> US09/178,973
<151> 1998-10-26
<160> 43
<210> 1
   <211> 24
   <212> DNA
   <213> Mus musculus
   <220>
   <400> 1
   agcactctcc agcctctcac cgca 24
<210> 2
   <211> 12
   <212> DNA
   <213> Mus musculus
   <220>
   <400> 2
   gatctgcggt ga 12
<210> 3
   <211> 24
   <212> DNA
   <213> Mus musculus
   <220>
   <400> 3
   accgacgtcg actatccatg aaca 24
<210> 4
   <211> 12
   <212> DNA
   <213> Mus musculus
   <220>
   <400> 4
   gatctgttca tg 12
<210> 5
   <211> 24
   <212> DNA
   <213> Mus musculus
   <220>
   <400> 5
   aggcaactgt gctatccgag ggaa 24
<210> 6
   <211> 12
   <212> DNA
   <213> Mus musculus
   <220>
   <400> 6
   gatcttccct cg 12
<210> 7
   <211> 1119
   <212> DNA
   <213> Mus musculus
   <220>
   <400> 7
   taaacaggct ctcctctcac ttatcaactg ttgacacttg tgcgatctct gatggctgtc 60
   ctgcagaaat ctatgagttt ttcccttatg gggactttgg ccgccagctg cctgcttctc 120
   attgccctgt gggcccagga ggcaaatgcg ctgcccgtca acacccggtg caagcttgag 180
   gtgtccaact tccagcagcc gtacatcgtc aaccgcacct ttatgctggc caaggaggcc 240
   agccttgcag ataacaacac agacgtccgg ctcatcgggg agaaactgtt ccgaggagtc 300
   agtgctaaag atcagtgcta cctgatgaag caggtgctca acttcaccct ggaagacgtt 360
   ctgctccccc agtcagacag gttccagccc tacatgcagg aggtggtacc tttcctgacc 420
   aaactcagca atcagctcag ctcctgtcac atcagcggtg acgaccagaa catccagaag 480
   aatgtcagaa ggctgaagga gacagtgaaa aagcttggag agagtggaga gatcaaggcg 540
   attggggaac tggacctgct gtttatgtct ctgagaaatg cttgcgtctg agcgagaaga 600
   agctagaaaa cgaagaactg ctccttcctg ccttctaaaa agaacaataa gatccctgaa 660
   tggacttttt tactaaagga aagtgagaag ctaacgtcca tcatcattag aagatttcac 720
   atgaaacctg gctcagttga aaaagaaaat agtgtcaagt tgtccatgag accagaggta 780
   gacttgataa ccacaaagat tcattgacaa tattttattg tcactgatga tacaacagaa 840
   aaataatgta ctttaaaaaa ttgtttgaaa ggaggttacc tctcattcct ttagaaaaaa 900
   agcttatgta acttcatttc catatccaat attttatata tgtaagttta tttattataa 960
   gtatacattt tatttatgtc agtttattaa tatggattta tttatagaaa cattatctgc 1020
   tattgatatt tagtataagg caaataatat ttatgacaat aactatggaa acaagatatc 1080
   ttaggcttta ataaacacat ggatatcata aaaaaaaaa 1119
<210> 8
   <211> 7445
   <212> DNA
   <213> Mus musculus
   <220>
   <400> 8 <210> 9
   <211> 1111
   <212> DNA
   <213> Mus musculus
   <220>
   <400> 9 <210> 10
   <211> 21
   <212> DNA
   <213> Mus musculus
<220>
   <400> 10
   ctgcctgctt ctcattgccc t 21
<210> 11
   <211> 21
   <212> DNA
   <213> Mus musculus
   <220>
   <400> 11
   caagtctacc tctggtctca t 21
<210> 12
   <211> 20
   <212> DNA
   <213> Mus musculus
   <220>
   <400> 12
   gacgcaagca tttctcagag 20
<210> 13
   <211> 16
   <212> DNA
   <213> Homo sapiens
   <220>
   <400> 13
   atgtatttcc,cagaaa 16
<210> 14
   <211> 17
   <212> DNA
   <213> Homo sapiens
   <220>
   <400> 14
   ccttttctgg gaaatac 17
<210> 15
   <211> 22
   <212> DNA
   <213> Mus musculus
   <220>
   <400> 15
   aggtgctcaa cttcaccctg ga 22
<210> 16
   <211> 22
   <212> DNA
   <213> Mus musculus
   <220>
   <400> 16
   ccactctctc caagcttttt ca 22
<210> 17
   <211> 21
   <212> DNA
   <213> Mus musculus
   <220>
   <400> 17
   caagtctacc tctggtctca t 21
<210> 18
   <211> 418
   <212> DNA
   <213> Homo sapiens
   <220>
   <900> 18
<210> 19
   <211> 21
   <212> DNA
   <213> Homo sapiens
   <220>
   <400> 19
   tggccaggaa gggcaccacc t 21
<210> 20
   <211> 21
   <212> DNA
   <213> Homo sapiens
   <220>
   <400> 20
   cctatcagat tgagggaaca g 21
<210> 21
   <211> 36
   <212> DNA
   <213> artificial sequence
   <220>
   <221> primer
   <222> 24,25,29,30,34,35
   <223> n is inosine in all cases
   <400> 21
   ggccacgcgt cgactagtac gggnngggnn gggnng 36
<210> 22
   <211> 20
   <212> DNA
   <213> artificial sequence
   <220>
   <400> 22
   ggccacgcgt cgactagtac 20
<210> 23
   <211> 20
   <212> DNA
   <213> Homo sapiens
   <220>
   <400> 23
   ccttccccag tcaccagttg 20
<210> 24
   <211> 20
   <212> DNA
   <213> Homo sapiens
   <220>
   <400> 24
   taattgttat tcttagcagg 20
<210> 25
   <211> 690
   <212> DNA
   <213> Homo sapiens
   <220>
<210> 26
   <211> 4797
   <212> DNA
   <213> Homo sapiens
   <220>
   <400> 26
   tgcacaagca gaatcttcag aacaggttct ccttccccag tcaccagttg ctcgagttag   60
   aattgtctgc aatggccgcc ctgcagaaat ctgtgagctc tttccttatg gggaccctgg  120
   ccaccagctg cctccttctc ttggccctct tggtacaggg aggagcagct gcgcccatca  180
   gctcccactg caggcttgac aagtccaact tccagcagcc ctatatcacc aaccgcacct  240
   tcatgctggc taaggaggta tacatctcaa tcctgctctt tctcgttgga tctacttgga  300
   atccaaatag ttcttaaact tttcttcaga gcatctctaa gagctttagg aacccactgt  360
   ttatccctga gggtagataa attttctgtt ttttcagaga ctctttggga atctggcttt  420
   ttttttttct tgaacttctt ccttccattt tggcctttat gatacatatg atgaattttt  480
   cccaaagagc ggccattcag taatccatct gatgattttt ttttccttta tgcctctgtg  540
   cattgttcta aactcatgca cacatctgaa ttctgctttt agtctttatg atgttgctct  600
   ggggagacgg gatggggcac atgtctatgt ataaattttt tttctatttg ctcaatgtcc  660
   agacccttag tcttttcttc tcttccaggc tagcttggct gataacaaca cagacgttcg  720
   tctcattggg gagaaactgt tccacggagt cagtgtaagc tacagttgtg acgaacaggg  780
   ccgtgtgccg tccatgggta cttggggtgg tggtgatgat ggtttaggtc ttatccctta  840
   tgaccctttc tgtttccctt ccacctgcag atgagtgagc gctgctatct gatgaagcag  900
   gtgctgaact tcacccttga agaagtgctg ttccctcaat ctgataggtt ccagccttat  960
   atgcaggagg tggtgccctt cctggccagg ctcagcaaca ggctaagcac atgtgtaagt 1020
   tcagctctca gcctatgccc acctacccct ccttccctcc ttccacagag acccccttac 1080
   cccaactctc tctccttccc cctaccccta agctagcagg aagaagtgtc ttggcagcag 1140
   tgttatcagg agtcatttgg gatcatagag tatttgcttt tgctttgact gagtcacatc 1200
   ttgagtttat agtggtgaat ggggtctgga acttaagtgt acagaagccg cattggtttg 1260
   tcttcggaaa aaaggcaact caggttgcgt aagatgagaa aggtgttggg aaaacatcta 1320
   gctgtggaaa tggatccatt gagtctaagt tgttgagggg aggggatggc atggagagaa 1380
   attagaagag aaagtgggaa atgggaaggc ttaaagtcgg tggtgggtcg gcagactgtt 1440
   gccctgttga tgtcatggga agccacaaaa tcggaggcgt gtgaacttga tgccgctgaa 1500
   catttgaaac tatgaaaaaa agtttgagtg gagtgggccc agtaaaaggc cctaggactt 1560
   actgaagagg gcttaatttt cacatgagat gttttatgta catttcttgt tctaagcatg 1620
   caattttctg gagatacgat tgaggtttta ttccttacag aatttgcata aactactccg 1680
   ctctttccac aaatgcaaac ctcagtagga tttcccaaag atgaagagag gtctcttgta 1740
   agggaagtga ctggattctg gcgtccaagg gaattcaaga gctcaggaaa tctaggtcac 1800
   tgttgaaatc taggtcattg tgggcaaaat tactaagagc tttaattcca ggtgaattgt 1860
   actgtacctc catgggtgtg gaggttcata aagtttcagc acaacattaa gatagttatg 1920
   cttgttattg ttttatagca tattgaaggt gatgacctgc atatccagag gaatgtgcaa 1980
   aagctgaagg acacagtgaa aaaggtagga ctgataactg tcaatgctaa gtcatgcaat 2040
   aggagagaca aatgttgttt ttctttcctt tctttcttcc catcactttg tgatttttca 2100
   cttgattctc ctaccaccag ggcgattact ttggtgtctg tgtatgtaga tatatctata 2160
   tatctagatg tcagtttcca aatcttgcaa attgtagaat tctagaactg gttgggatct 2220
   tagcttgtct agtcacataa cctcagattc tggggatggt cagtggcaga gatagggcta 2280
   gaatgcaggt ctcctgaatc ccaagccagc acttttcccg gtggtgatac agattagttt 2340
   tggtaccatt aattcttagg gaaatttcag attcctattg actcatgtaa tctgaagaag 2400
   tacttgttta aaaacagaaa aatgcctatg ggcaaattta tttgaagtca tttttgaagt 2460
   cattaatgca ttgctttgaa acttggaaga ataaactcag aacaatgaga aaagagctgg 2520
   acttgcatat agggctaatt tctggagtaa taaacactta ttttgaatta tcataatatc 2580
   tatcagatat tgattatagt ttaaaagcaa gagcagacaa ccccgatctc ttttatacag 2640
   gttcaaatag agtaaaaata ttagtaagag atttattata gttaaatgga agtctgaatt 2700
   ggtaagcttt tttttcttcc tctctcccat caagaccttc cattctagtt tcttccttca 2760
   ctccctcaac aaatccctag ggagcattta tccatggtgg gctggtgtac atttctatag 2820
   tgaatgatac catcatgtgg cctatttggt gaaaagaaca acaatggaag gcttagacta 2880
   acaatagtga ctcaccccaa aaccggagga atgattagga gcagtgaaag tgacgctctt 2940
   gcaagcaggt acaactaaat actcagaaac atgaaggctc cagttgatgg aattttcagt 3000
   aacaagctta accttaattc cccctttttc cctcttgact ttttaaaaaa gcgtttcttc 3060
   ctgagcatca tttaatgagt gtgactgttt cttcctttga taattgaagg ctttgtagtt 3120
   ttaaattgtg aagcccagtt ctcttgttat agaactatta tctagacatg gagggctgaa 3180
   tgttagcatg ccacagacaa ggcatgcttt acacatcttg cttaaaaaat tactgatttc 3240
   atcttgcttg ttgtctttag aaaagtgaag tgtgagagag gagaatctca tggtgatctg 3300
   tgtgattttc aagaccttta atccattttg aaagaatcaa tttcatattt gcaatgggtt 3360
   gccatgtgga agagtgatta tgcttttttg ctggtagctt cagaaagcac aggagggaga 3420
   gcaatgttgt tcagagaaag atcaacagga ggagaaactg tcagagctgt ctgaaatagg 3480
   gtggttttgg gaggcattaa ttccctctcg ttgggggtaa aagcagaacg caggttggta 3540
   gtaaaatgca tgacagacag taggggacga taaactttaa aattctttat agtcttggag 3600
   tctttgagat agaaaagaat atctttttgg ccttatgtca aaagaagtat ggaaaggtga 3660
   aagggcggaa gaaagcagga aaaggaagaa ccatgtatta tatagaggac aatggtgaca 3720
   aggtttttct tgaaataatg caaatatgat agattagagg aatttcagta gggaatgctt 3780
   ttcacttgaa tttgggtttc ctcttcgatt aagtttggga tcctcatctg catttgactt 3840
   ggagagagaa agaatgaatg ttaggaccta tatctggttt tctattaact aaagcaagtg 3900
   gaaaagactt atttggtatt tttcccacaa aagtgaaaac ttttctttta ctgtttgtca 3960
   aaaaggtgga aatagaaaaa gccttaatgt attggtgaat acatggttca aagtcatttg 4020
   agtagagatg ttttaaatca ggagtgtcca atcatttggc ttccctggac caccttgaaa 4080
   gaattgtctt ggtacacaca taaaatacaa gaacaatagc tgatgagcta aaaaagtcca 4140
   tgcataaatc tcatactgtt ttaagaaagt ttatgaattt ctgttagggt gcattcaaag 4200
   ctgtcctggg ccatgtgcgg cctgtgggct gcaggttgga caagctcctt ataagtaatc 4260
   tgtcatagat agttttggag ctgcaaaaca ggccaaggca taatgggtgg cactcgggat 4320
   cccccagatc ccagcctcac ttcagtctcc ttgctctggt taagaagggg tggtcaactc 4380
   tctgcccagc ttttaaacag cttcattagt gtgaggtgca cctgaaattg atgcctgctg 4440
   gtggcctctc agtccagaga gccgtcattt taagctcttt ggcaaatcat acaatactaa 4500
   agggatatta ctatgaatgt tttacaaatg cttaaaactc ggtttctgtc tccatcaacc 4560
   taatcttgca atttctaatt tgttcacttt agaaaacatg gcataaatgc tcaaatactt 4620
   ttgcattctt attttcacag cttggagaga gtggagagat caaagcaatt ggagaactgg 4680
   atttgctgtt tatgtctctg agaaatgcct gcatttgacc agagcaaagc tgaaaaatga 4740
   ataactaacc ccctttccct gctagaaata acaattagat gccccaaagc gattttt 4797
<210> 27
   <211> 20
   <212> DNA
   <213> Homo sapiens
   <220>
   <400> 27
   atcagatgga ttactgaatg 20
<210> 28
   <211> 20
   <212> DNA
   <213> Homo sapiens
   <220>
   <400> 28
   agctcagcta cagcacagat 20
<210> 29
   <211> 20
   <212> DNA
   <213> Homo sapiens
   <220>
<400> 29
   cctgccccat ttattggcag 20
<210> 30
   <211> 20
   <212> DNA
   <213> Homo sapiens
   <220>
   <400> 30
   tgtcctctgc caccctaaca 20
<210> 31
   <211> 20
   <212> DNA
   <213> Homo sapiens
   <220>
   <400> 31
   taattcacca ggaccatcat 20
<210> 32
   <211> 20
   <212> DNA
   <213> Homo sapiens
   <220>
   <400> 32
   gtggactcag gcaatgatgt 20
<210> 33
   <211> 20
   <212> DNA
   <213> Homo sapiens
   <220>
   <400> 33
   acatagagtg ttaaagtggg 20
<210> 34
   <211> 20
   <212> DNA
   <213> Homo sapiens
   <220>
   <400> 34
   gctggaaggt ggacagcgag 20
<210> 35
   <211> 20
   <212> DNA
   <213> Homo sapiens
   <220>
   <400> 35
   tggcatcgtg atggactccg 20
<210> 36
   <211> 20
   <212> DNA
   <213> Mus musculus
   <220>
   <400> 36
   tctgctccct gctcctggga 20
<210> 37
   <211> 20
   <212> DNA
   <213> Mus musculus
   <220>
   <400> 37
   tccaggaggt ctgtagtaat 20
<210> 38
   <211> 21
   <212> DNA
   <213> Mus musculus
   <220>
   <400> 38
   ctgcctgctt ctcattgccc t 21
<210> 39
   <211> 21
   <212> DNA
   <213> Mus musculus
   <220>
   <400> 39
   caagtctacc tctggtctca t 21
<210> 40
   <211> 179
   <212> PRT
   <213> Mus musculus
   <220>
   <400> 40
   Met Ala Val Leu Gln Lys Ser Met Ser Phe Ser Leu Met Gly Thr Leu
   1               5                   10                  15
   Ala Ala Ser Cys Leu Leu Leu Ile Ala Leu Trp Ala Gln Glu Ala Asn
               20                  25                  30
   Ala Leu Pro Val Asn Thr Arg Cys Lys Leu Glu Val Ser Asn Phe Gln
           35                  40                  45
   Gln Pro Tyr Ile Val Asn Arg Thr Phe Met Leu Ala Lys Glu Ala Ser
       50                  55                  60
   Leu Ala Asp Asn Asn Thr Asp Val Arg Leu Ile Gly Glu Lys Leu Phe
   65                  70                  75                  80
   Arg Gly Val Ser Ala Lys Asp Gln Cys Tyr Leu Met Lys Gln Val Leu
                   85                  90                  95
   Asn Phe Thr Leu Glu Asp Val Leu Leu Pro Gln Ser Asp Arg Phe Gln
               100                 105                 110
   Pro Tyr Met Gln Glu Val Val Pro Phe Leu Thr Lys Leu Ser Asn Gln
           115                 120                 125
   Leu Ser Ser Cys His Ile Ser Gly Asp Asp Gln Asn Ile Gln Lys Asn
       130                 135                 140
   Val Arg Arg Leu Lys Glu Thr Val Lys Lys Leu Gly Glu Ser Gly Glu
   145                 150                 155                 160
   Ile Lys Ala Ile Gly Glu Leu Asp Leu Leu Phe Met Ser Leu Arg Asn
                   165                 170                 175
   Ala Cys Val
<210> 41
   <211> 179
   <212> PRT
   <213> Mus musculus
   <220>
   <400> 41
   Met Ala Val Leu Gln Lys Ser Met Ser Phe Ser Leu Met Gly Thr Leu
   1               5                   10                  15
   Ala Ala Ser Cys Leu Leu Leu Ile Ala Leu Trp Ala Gln Glu Ala Asn
               20                  25                  30
   Ala Leu Pro Ile Asn Thr Arg Cys Lys Leu Glu Val Ser Asn Phe Gln
           35                  40                  45
   Gln Pro Tyr Ile Val Asn Arg Thr Phe Met Leu Ala Lys Glu Ala Ser
       50                  55                  60
   Leu Ala Asp Asn Asn Thr Asp Val Arg Leu Ile Gly Glu Lys Leu Phe
   65                  70                  75                  80
   Arg Gly Val Ser Ala Lys Asp Gln Cys Tyr Leu Met Lys Gln Val Leu
                   85                  90                  95
   Asn Phe Thr Leu Glu Asp Ile Leu Leu Pro Gln Ser Asp Arg Phe Arg
               100                 105                 110
   Pro Tyr Met Gln Glu Val Val Pro Phe Leu Thr Lys Leu Ser Asn Gln
           115                 120                 125
   Leu Ser Ser Cys His Ile Ser Gly Asp Asp Gln Asn Ile Gln Lys Asn
       130                 135                 140
   Val Arg Arg Leu Lys Glu Thr Val Lys Lys Leu Gly Glu Ser Gly Glu
   145                 150                 155                 160
   Ile Lys Ala Ile Gly Glu Leu Asp Leu Leu Phe Met Ser Leu Arg Asn
                   165                 170                 175
   Ala Cys Val
<210> 42
   <211> 5935
   <212> DNA
   <213> Mus musculus
   <220>
   <400> 42
   gaattcaagt ccacatgcaa tcaatccgaa tactttgtaa attctcttct tcaaatatcc 60
   atctatatag tataagttat tgtaggatca tttaaaaata atgttttgag acttatgttt 120
   gcacaagtaa aatgtcagag agaattagca aatgtatagt attattttat tttaaaaaat 180
   ctatgcttaa aatgtctatt agattgttca ctactgacat ttccaaactt aacttgacct 240
   tggctatgat ttcaaccttt gtatttgcat ctaccataac tgtgtgctca cttaccatgc 300
   tatccgacga gcatgttccc ctgatgtttt tgccttttgc tctctcgcta acaggctctc 360
   ctctcagtta tcaacttttg acacttgtgc gatcggtgat ggctgtcctg cagaaatcta 420
   tgagtttttc ccttatgggg actttggccg ccagctgcct gcttctcatt gccctgtggg 480
   cccaggaggc aaatgcgctg cccatcaaca cccggtgcaa gcttgaggtg tccaacttcc 540
   agcagccgta catcgtcaac cgcaccttta tgctggccaa ggaggtacag ctgcatctct 600
   ttctctccat accgccttgc catttctctg aagcacttgc aaactcttta ggggcgcttt 660
   atctccgcag gtctcactac ctatgttttc tgtctcttta gagactcttt aaggactgga 720
   tctttttcta tttctatttc aaggtctcag gaccatttcc tatcttggcc ttcaggacac 780
   atatactgaa ttttatctac agaggcgcgt ttagaaagcc acccacgact gcaatacttt 840
   ccatcctgtt gtgctctctt ctgaactcat actctcttgg ctactcctga gacccactgc 900
   ggacatacat ctctacttac aggcttttct tccatctcct tgtcacccag gcacttaggg 960
   ttttctctct ttcaggccag ccttgcagat aacaacacag acgtccggct catcggggag 1020
   aaactgttcc gaggagtcag tgtaagtcct cactgtgatg agcagggcta gctgcgggag 1080
   ctggtggacc ctctgggata gtctgacgta tgacccctgc tgcttcttgt ctacctgcag 1140
   gctaaggatc agtgctacct gatgaagcag gtgctcaact tcaccctgga agacattctg 1200
   ctcccccagt cagacaggtt ccggccctac atgcaggagg tggtgccttt cctgaccaaa 1260
   ctcagcaatc agctcagctc ctgtgtaagt ctggctctgg ctacctatgc tcctctctct 1320
   tcctcttcta ttccagtaag aacccgaggt cctgccctct ctctcttcac aagagtgagg 1380
   agggcctcag caccaccacc atcataggcc acttgaaata ggtcacaaag gctttggctt 1440
   caattgagta atactttgag tttgtattag ttaagcttta tttgttttat ccatggaaag 1500
   aaatcaactc aaattctgta ggatgagaaa gatgttggga acgaaaaaag gcctagatag 1560
   agaaacagat ctgctgagta cagtacttat gggggggggg ggcagggggc gatatccact 1620
   gagtccaagt acttgttggg agagaaatcc actgagtaca agtacttgtg ggggaaggaa 1680
   tggcacagag caaaagttga agggaaagag gaagatggag aggcctcaat gttgggggtg 1740
   tgaaaggtca ctcctttttc catgtgatgg agagttaaga aaaatcagtg tgtgagtttg 1800
   atgtcttcag acaccccaac tatggcagac tgtgggagac ctggcattta gggaaggcgc 1860
   ggcttttcac acgagaaact ttatgctcat ctcttgtgct acactcccac ctttgatgag 1920
   gttaagctca ggtttcgttt ctaccgttct tgctactggt ggaaacttca gtaggattcc 1980
   ccaaagacga ggacagctct tctgtaaggg agggacctgg atttcagtgt cctagagaac 2040
   gaaatagctc agagaatcta ggtcaacgtg aaatctaggt cacagcgggc aaaaatgact 2100
   gaacgcctct attccaggtg aacggtcacg tgcctcagat atactgaggt attgggctcc 2160
   caccggataa gattctgtta gtgagtctgc ttttattttg cagcacatca gtggtgacga 2220
   ccagaacatc cagaagaatg tcagaaggct gaaggagaca gtgaaaaagg tactattggc 2280
   aagccacaat actaagccat tcagtaggag acgtggggat ttctttctct gcttcccagt 2340
   ctcttctact ttgtaacatt ttctttgact tgtctactgt ctggtccatt actcacttag 2400
   ctgcacctgc atctagctgg gtctatagat ctttcaatct gtgtctaaat ttgtaagtca 2460
   caattctgga gctagcagaa agcttagctc agccagtctc atgagcactt gctcggagga 2520
   tggcttgtga cagagtcaat gctagaagac agcatccctg attcccagct ctgcacttgc 2580
   ctagtggcca cgtgtaatta ctttagcctg attaagtatt tgggaaagcc aattcccacc 2640
   gacctacata atccgaagaa gcatgcattg aaaactagaa agctgggcac aaacttacta 2700
   gagatgattt ttgagctcat taaactgatg ctctgaaatg tgatcaaatc aacccagaat 2760
   aacaacaaaa gagctggatt tgcaaatagg acaagtattt agaatcactg gtattaacag 2820
   ctgtcatctt aattaaaata tagtgtctat ttagctgcct atttaagatt aaacacaaga 2880
   gtggataact tcccaattta ctgggcctgg tttcaataga gtaaaaatat cagtcataga 2940
   ttaattatag tgtcatgaaa gtatgagttg gaaacccttt ccttactttt taccttcatt 3000
   tcttagttat tatttttttt tcttcacacc ctgatcaagc cactagtaag cacctatctg 3060
   ctgcgagcta ttatatgact ttacagcaaa caacattgct gtgtggcctc tttggggaag 3120
   ggaacaggat agcaggaggc tcaggctagc aagtctggac tcaacctaaa gccagaggca 3180
   tggttgatag cagagaaagt gaggctcttc acaagtgggt gtgcttaagt aatcagaaac 3240
   aggaaggctc tggttgatgg aattatcagt aagatatcta cccttatctc cttcttctat 3300
   agaagctaaa ccgtctctcc ttcttgtgtg taggctgata aacacgcttg ttttcttttg 3360
   agtgttcatg gctttgcaga ttttcagtgc tctgccagtt cttgttagag ggtttgttac 3420
   cttgacacct gggcttggat gttagcatgc caaaggcaca cacttctgaa tgcctgtgta 3480
   aaaggttatt attcatttac tttgtctttg gaaaggtgaa gtgtgtgtga gaaagaactc 3540
   acaggagatg tattctctgt aggaaaactt ttttttcccc ttaaaagcct ataatccact 3600
   ttcagtcaac tttgactttt ataccatgct gtcacatgaa agagtgttta ggcccgctct 3660
   cgtggctctg ggaaaagcac caatagggga agaaatgtta tgccgagaaa tctgactggc 3720
   agggaaactg ggtcagagct ccccaaagac cactacaggt gttaagtagg aacagtcgag 3780
   ggtgggttca tataatagaa tggaacagag ggagggaaga taagctacaa agtttcatag 3840
   ggtcctaagt ctttaagata caaaatagct ggttgggctt cataacaaag gaagtctggg 3900
   aaggcagcaa gcattgagag ggagatggaa agggaaaaaa caatgtagag gatttgaaaa 3960
   gctacaaatc ctccacgaga ggatttttct tggaggaatc tagaacaagg gtggtggatt 4020
   aggtggatcg cagaaggact tgctttgcca tttgaatctg ggtttttgtc tctccattga 4080
   ggttgagagc gtcacccttt tttaccctgg ataggaggag gaaagaaggg gtgttttgac 4140
   tcctacctgg agttttacta gtttacgcaa tggaacagac actcgggacc tcctcttgac 4200
   aagaaaaaaa aaaaaaaaag gaaacctgtt gtttctcttg tttgttcttt tgttaagaaa 4260
   gcacaggcag ctgggcatgg tggcccatgc ctttaatccc agcatttggg aggcagaggc 4320
   aggtgacttt ctaaattcaa ggccagcctg gtctacaaag tgagttccag gacagccagg 4380
   gctatacaga gaaaccctgt ctcgggaaaa aaaaaaaaga agaaaagaaa agaaaagaag 4440
   agaagaggag aggagaggag aggagaggag aggagaggag aggagaggag aggagaggag 4500
   aggagaggag aagagaagag aagagaagag aagagaagag aagagaagag aagagaagag 4560
   aagagaagag aagagaagag aagagaagag aagagaagag aagagaaaag aaaagagaaa 4620
   agaaaagaaa aaagcaagca agcaagcact ggcaaagcat gcccacatgg gacgtatgtg 4680
   ggtctttgag acaaggcttt tgaattgagc gctcatcaat agttgatcat ggtcaggtgg 4740
   agggctacct gtcaggccga gccctgctgg cttagcactt aacatctcca ggtctcagta 4800
   tcacttcctg ctgcttagca cagttaggag ttgagcaaac ctttttttcc aacccccact 4860
   aaaatttaat ttacaaaagg cagtgtaatt tgtgggatac agtgtgataa ttgatctatg 4920
   tgtgcattgt gcaaggttca ataaggtaga tcaataggcc catcaacagc tttatgggtg 4980
   tgaaatgcaa gtaatatagg tagatgcctg tgtgtcctta ggtcagaaag gcatgatttt 5040
   aaggtcttgg gcaaatcata ttatactcat gttaaaaatg cattatgttg attatcaatc 5100
   ttttagagaa ggctgatact tggttttggt gctcagcaag caaatgtcac cagctctttc 5160
   taactagtac cactttagaa aatgctaccc gtgctcaaat tggtttgtat tcttattttc 5220
   atagcttgga gagagcggag agatcaaagc gatcggggaa ctggacctgc tgtttatgtc 5280
   tctgagaaat gcttgcgtct gagcgagaag aagctagaaa acgaagaact gctccttcct 5340
   gccttctaaa aagaacaata agatccctga atggactttt ttactaaagg aaagtgagaa 5400
   gctaacgtcc accatcatta gaagatttca catgaaacct ggctcagttg aaagagaaaa 5460
   tagtgtcaag ttgtccatga gaccagaggt agacttgata accacaaaga ttcattgaca 5520
   atattttatt gtcattgata atgcaacaga aaaagtatgt actttaaaaa attgtttgaa 5580
   aggaggttac ctctcattcc tctagaagaa aagcctatgt aacttcattt ccataaccaa 5640
   tactttatat atgtaagttt atttattata agtatacatt ttatttatgt cagtttatta 5700
   atatggattt atttatagaa aaattatctg atgttgatat ttgagtataa agcaaataat 5760
   atttatgata ataactatag aaacaagata tcttaggctt taataaacac atgaatatca 5820
   taaatcttct gtcttgtaat ttttctccct ttaatatcaa caataccatc atcgtcatca 5880
   ttacccaatc attctcatga cttcatgctt gactcatatt atctggtaaa gtttg      5935
<210> 43
   <211> 179
   <212> PRT
   <213> Homo sapiens
   <220>
   <400> 43
   Met Ala Ala Leu Gln Lys Ser Val Ser Ser Phe Leu Met Gly Thr Leu
   1               5                   10                  15
   Ala Thr Ser Cys Leu Leu Leu Leu Ala Leu Leu Val Gln Gly Gly Ala
               20                  25                  30
   Ala Ala Pro Ile Ser Ser His Cys Arg Leu Asp Lys Ser Asn Phe Gln
           35                  40                  45
   Gln Pro Tyr Ile Thr Asn Arg Thr Phe Met Leu Ala Lys Glu Ala Ser
       50                  55                  60
   Leu Ala Asp Asn Asn Thr Asp Val Arg Leu Ile Gly Glu Lys Leu Phe
   65                  70                  75                  80
   His Gly Val Ser Met Ser Glu Arg Cys Tyr Leu Met Lys Gln Val Leu
                   85                  90                  95
   Asn Phe Thr Leu Glu Glu Val Leu Phe Pro Gln Ser Asp Arg Phe Gln
               100                 105                 110
   Pro Tyr Met Gln Glu Val Val Pro Phe Leu Ala Arg Leu Ser Asn Arg
           115                 120                 125
   Leu Ser Thr Cys His Ile Glu Gly Asp Asp Leu His Ile Gln Arg Asn
       130                 135                 140
   Val Gln Lys Leu Lys Asp Thr Val Lys Lys Leu Gly Glu Ser Gly Glu
   145                 150                 155                 160
   Ile Lys Ala Ile Gly Glu Leu Asp Leu Leu Phe Met Ser Leu Arg Asn
                   165                 170                 175
   Ala Cys Ile
<110> Dumoutier, Laure Renauld, Jean-Christophe
<120> Isolated Nucleic Acid Molecules which Encode T Cell Inducible Factors, or Interleukin-21, The Proteins Encoded, and Uses Thereof
<130> LUD 5664
<140>
   <141>
<150> US09/419,568
   <151> 1999-10-18
<150> US09/354,243
   <151> 1999-07-16
<150> US09/178,973
<151> 1998-10-26
<160> 43
<210> 1
   <211> 24
   <212> DNA
   <213> Mus musculus
   <220>
   <400> 1
   agcactctcc agcctctcac cgca 24
<210> 2
   <211> 12
   <212> DNA
   <213> Mus musculus
   <220>
   <400> 2
   gatctgcggt ga 12
<210> 3
   <211> 24
   <212> DNA
   <213> Mus musculus
   <220>
   <400> 3
   accgacgtcg actatccatg aaca 24
<210> 4
   <211> 12
   <212> DNA
   <213> Mus musculus
   <220>
   <400> 4
   gatctgttca tg 12
<210> 5
   <211> 24
   <212> DNA
   <213> Mus musculus
   <220>
   <400> 5
   aggcaactgt gctatccgag ggaa 24
<210> 6
   <211> 12
   <212> DNA
   <213> Mus musculus
   <220>
   <400> 6
   gatcttccct cg 12
<210> 7
   <211> 1119
   <212> DNA
   <213> Mus musculus
   <220>
   <400> 7
   taaacaggct ctcctctcac ttatcaactg ttgacacttg tgcgatctct gatggctgtc 60
   ctgcagaaat ctatgagttt ttcccttatg gggactttgg ccgccagctg cctgcttctc 120
   attgccctgt gggcccagga ggcaaatgcg ctgcccgtca acacccggtg caagcttgag 180
   gtgtccaact tccagcagcc gtacatcgtc aaccgcacct ttatgctggc caaggaggcc 240
   agccttgcag ataacaacac agacgtccgg ctcatcgggg agaaactgtt ccgaggagtc 300
   agtgctaaag atcagtgcta cctgatgaag caggtgctca acttcaccct ggaagacgtt 360
   ctgctccccc agtcagacag gttccagccc tacatgcagg aggtggtacc tttcctgacc 420
   aaactcagca atcagctcag ctcctgtcac atcagcggtg acgaccagaa catccagaag 480
   aatgtcagaa ggctgaagga gacagtgaaa aagcttggag agagtggaga gatcaaggcg 540
   attggggaac tggacctgct gtttatgtct ctgagaaatg cttgcgtctg agcgagaaga 600
   agctagaaaa cgaagaactg ctccttcctg ccttctaaaa agaacaataa gatccctgaa 660
   tggacttttt tactaaagga aagtgagaag ctaacgtcca tcatcattag aagatttcac 720
   atgaaacctg gctcagttga aaaagaaaat agtgtcaagt tgtccatgag accagaggta 780
   gacttgataa ccacaaagat tcattgacaa tattttattg tcactgatga tacaacagaa 840
   aaataatgta ctttaaaaaa ttgtttgaaa ggaggttacc tctcattcct ttagaaaaaa 900
   agcttatgta acttcatttc catatccaat attttatata tgtaagttta tttattataa 960
   gtatacattt tatttatgtc agtttattaa tatggattta tttatagaaa cattatctgc 1020
   tattgatatt tagtataagg caaataatat ttatgacaat aactatggaa acaagatatc 1080
   ttaggcttta ataaacacat ggatatcata aaaaaaaaa 1119
<210> 8
   <211> 7445
   <212> DNA
   <213> Mus musculus
   <220>
   <400> 8
   gtctatcact tgcttaagat tcttctaatt tataaaaaaa actatttctt aaaatgaaaa   60
   gcaaacagag cacgtattta tagcatggtg ttctgaccat gcaggtacag agtggaatgg  120
   taagaggcgc tattatcagc attaaccaac atgttaatgt tttcttctgg caagcaaact  180
   tgaaatctat gtcttaaaca atcttcaagc ctctaatata gtgctaacga ctggagtccg  240
   ctgctgtcca acagagctct tgagcacgct ctcctctgtt tgcaatttta tgttctttga  300
   tcgactcccc aacctctcac cttcggctcc tgatggccac ctttcaactt tctgcattta  360
   tgaactccat gttttaatct ttttattaaa atattcacac aatcagtgtt tgtgcaagtc 420
   tgtttcaccc acatgtatgt ctgtgcacca agtgctgcct ggtgcttgtg ggggcaagga 480
   gcaggagagg gtgccctggc accggagtca cggatggttg tgagccacca tgaggatgct 540
   gggagttaga cccaggtcct ccagaagtgc agcaaatgct cttaaccaca cgcaggcatt 600
   tctctctcca gccccaacat gagtgctttt agattccacc tagaatagag atctgatggc 660
   ttcactcact gccacctccc ctttgcatct ttctgccaag gaacaccaaa aagcaagaat 720
   ccccacactg ctttcgctcc tcaagtctgc acctctcaac aggtcaagat tctccagtgt 780
   ccctctaaca ctttccccag tgtccctcta acactttctc cagtgtccct ctaacacttt 840
   ctccagtgtc cctctaacac ttttgatctc aattagctga ggggagaaag atctcacaca 900
   gtgattttca tgacttcgcg ttctagtcta gatgtaggca tttgcgtgtc agtctagggt 960
   aggcgtctgc tcccgctgct taggaaagac tttcctagtc tagttgtcag gtgctatctg 1020
   ggattcagtg tacatacaat gcaaaaaatc ccagtatttt gtaaattctc ttcttcaact 1080
   atccatctat atagtatgtt attgtaggct catttaaaaa taatattttg agacttatgc 1140
   ttgcacaagt aaaatgtcag agaattagca aatgtatagt attattttat tttaaaaaaa 1200
   tctatgctta aaatgtctat tagattgttc actaccgata tttccaaact taacttgacc 1260
   ttggctatga tttcaacctt tgtatttgca tctaccataa cagtctctga accagaacat 1320
   tctgtggcaa tgggagctgt gaagaaagcc aacattctta ttaaaaaaaa aaaacagcta 1380
   gttatagttt aggattccat atactaaaaa aaatagagat ataattattt taaaaattga 1440
   aataatctcc aagttttcat tatggcttat ttcaaagcac agaatatagg acacgggtct 1500
   tttatttctg gtcacttcta aagagataag aatctatgaa gttggtggga aaatgagtcc 1560
   gtgaccaaaa cgctgactca atagctacgg gagatcaaag gctgctctac tcaatcagaa 1620
   tctactacgg caaagccatg gctttctttg aaaaccgtgt ttagaagatt tctgggattt 1680
   gtgtgcaaaa gcaccttgtt ggccctcacc gtgacgtttt agggaagact tcccatctct 1740
   caaggtggga aggcttggag gtggtgtctt gtggcctcct atggtggtta ggtacttctc 1800
   agaagacagg actggaaatt agataatgtc tgatgtcata tcattcacaa taccaaaaaa 1860
   accctggtgt cccgatggct ataaaagcag caacttctgc ctctcccatc acaagcagag 1920
   acacctaaac aggtaagcac tcagacctct acagacaatc atctgcttgg taccatgcta 1980
   cccgacgaac atgctcccct gatgtttttg ccttttgctc tctcactaac aggctctcct 2040
   ctcacttatc aactgttgac acttgtgcga tctctgatgg ctgtcctgca gaaatctatg 2100
   agtttttccc ttatggggac tttggccgcc agctgcctgc ttctcattgc cctgtgggcc 2160
   caggaggcaa atgcgctgcc cgtcaacacc cggtgcaagc ttgaggtgtc caacttccag 2220
   cagccgtaca tcgtcaaccg cacctttatg ctggccaagg aggtacagct gcatctcttt 2280
   ctctccatac cgccttgcca ttttctctga agcacttgca aactctttag gggcgcttta 2340
   tctccgcagg tctcactacc tatgttttct gtctctttag agactcttta aggactgggt 2400
   ctttttctat ttctatttca aggtctcagg accatttcct atcttggcct tcaggacaca 2460
   tatactgaat tttatctaca gaggcgcatt tagaaagcca cccacgactg caatactttc 2520
   catttctctg tgctctcttc tgaactcata ctctcttggc tactcctgag acccactgcg 2580
   gacatacatc tctacttaca ggcttttctt ccatctcctt gtcacccagg cacttagggt 2640
   tttctctctt tcaggccagc cttgcagata acaacacaga cgtccggctc atcggggaga 2700
   aactgttccg aggagtcagt gtaagtcctc actgtgatga gcagggctag ctgcgggagc 2760
   tggtggaccc tctgggatag tctgacgtat gacccctgct gcttcttgtc tacctgcagg 2820
   ctaaagatca gtgctacctg atgaagcagg tgctcaactt caccctggaa gacgttctgc 2880
   tcccccagtc agacaggttc cagccctaca tgcaggaggt ggtacctttc ctgaccaaac 2940
   tcagcaatca gctcagctcc tgtgtaagtc tgactctggc tacctatgct cctctctctt 3000
   cctcttctat tccagtaaga acccgaggtc ctgccctctc tctcttcaca agagtgagga 3060
   gggcctcagc accaccacca tcataggcca cttgaaatag gtcacaaagg ctttggcttc 3120
   aattgagtaa tactttgagt ttgtatgagt gaagctttat ttgttttatc catggaaaga 3180
   aatcaactca aattctgtag gatgagaaag atgttgggaa cgaaaaaagg cctagataga 3240
   gaaacagatc tgctgagtat agtacttatg gggggagcag ggggcgatat ccactgagta 3300
   caagtacttg tggggagaga aatccactga gtacaagtac ttgttggcat ggagatccac 3360
   tgagtacaag tacttgtggg gggagggaat ggcacagagc aaaagttgaa gggaaggaag 3420
   atggagaggc ctcatggttg ggggtgtgaa aggtcactcc ttttccatgt gatggagagt 3480
   taagaaaaac cagtgtgtga gtttgatgtc ttcagacacc cccaactatg aaacatatcc 3540
   acgaggagcg ggcagactgt gggagacctg gcatttaggg aaggcgcggc ttttcacacg 3600
   agaaacttta tgctcatctc ttgtgctaca ctcccacctt tgatgaggtt cagctcaggt 3660
   ttcgtttcta ccgttcttgc tactggtgga aacttcagta ggattcccca aagacgagga 3720
   cagctcttct gtaagggagg gacctggatt tcagtgtcct agagaacgaa atagctcaga 3780
   gaatctaggt caacgtgaaa tctaggtcac agcgggcaaa aatgactgaa cgcctctatt 3840
   ccaggtgaac ggtcacgtgc ctcagatata ctgaggtatt gggctcccac cggataagat 3900
   tctgttagtg agtctgcttt tattttgcag cacatcagcg gtgacgacca gaacatccag 3960
   aagaatgtca gaaggctgaa ggagacagtg aaaaaggtac tattggcaag ccacaatact 4020
   aagccattca gtaggagacg tggggatttc tttctctgct tcccagtccc ttctactttg 4080
   taacatttta tttgacttgt ctactatctg gtccattact cgcttagctg cacctgtatc 4140
   tagctgggtc tatagatctt tcaatctgtg tctaaatttg taagtcacaa ttctggagct 4200
   agcagaaagc ttagctcagc cagtctcatg agcacttgct cggaggatgg cttgtgacag 4260
   agtcaatgct agaagacagc atccctgatt cccagctctg cacttgccta gtggccatgt 4320
   gtaattactt tggcttgatt aagtatttgg gaaagccagt tcccacggac ctacataatc 4380
   tgaagaacca tgcattgaaa actagaaagc tgggcacaaa cttactagag atgatttttg 4440
   agctcattaa acggatgctc tgaaatgtgg caaaatcaac ccagaataac aacaaaagag 4500
   ctggatttgc aaataggaca agtatttaga atcactggta ttaatagcta tcatcttaat 4560
   taaaatatag ggcctatata tatatttaag attaaacaca agagtggata gcctcccaat 4620
   ttacttggcc tggtttcaaa agagtaaaaa tatcagtcat ggattaatta tagtgtcatg 4680
   aaagtatgag atggaaaccc tttccttact ttttaccttc atttcttagt tttttttttc 4740
   ttcacaccct gatcaagcca ctagtaagca cctatctgct gtgagctatt atatgacttt 4800
   acagcaaaca acattgctgt gtggcctctt tggggaaggg aacaggatag caggaggctc 4860
   aggctagcaa gtctgacttg ccctaaagcc agaggcatgg ttgatagcag agaaagtgag 4920
   gctcttcgca agtgggtgtg cttaagtaat cagaaacagg aaggctccgg ttgatggaat 4980
   tatcagtaag atatctaccc ttatctcctt ctatcgaacc taaatcgtct ctttttcttg 5040
   tgtgtaggct gataaacaca cttgttttct tttgagtgtt catggctttg tagattttta 5100
   gtgctctgcc agttcttgtt agagggtttg ttaccttgac acctgggctt ggatgttagc 5160
   atgccaaagg cacacacttc tgaatgcctg tgtaaaaggt tattattcat ttactttgtc 5220
   tttggaaagg tgaagcgtgt gtgagaaaga actcacagga gatgtgttct ctgtaggaaa 5280
   actttttttt tccccttaaa tgcctataat ccactttcag tcaactttga cttttatacc 5340
   atgctgtcac atgaaagagt gtttaggccc gctctcatgg ctctgggaaa agcaccaata 5400
   ggggaaggaa tgttatgctg agaaatctga ccggcaggga aactggtcag agctcccccg 5460
   aagaccacca caggtgttaa gtaggaacag tccagggtgg gctcatgtaa tagaatggaa 5520
   cagagcgagg gaagataagc tacaaagttt catagggtcc ggagtcttaa agatacaaaa 5580
   tagctgcttg ggcttcataa caaaggaagt ctgggaaggc agcaagtgag agggaaatgg 5640
   aaagggaaaa aacagaatgt agaggacttg aacagctaca aatcctctac cagacgattt 5700
   ttcttggaac aatctagaag gtagtggatt aggtgattgc agggggactt gctttgccat 5760
   ttgaatctgg gtttttgtct ctccattgag gttgaaagcg tcaccctttt taccctcgaa 5820
   tggaggagga aagaaggggt gttatgactc ctacctggag ttttactagt ttacgcaatg 5880
   gaacagacac tcgggacctc ctcttgacaa aaaaaatgga aacctgttgt ttgtcttgtt 5940
   tgttcttttg ttaagaaagc acaggcaaag cccgaccaca tgggttgaat gtgggtcttt 6000
   gagtcaaggc ttttgagttg agcactcatc aatagttgat catggtcagg tggagggcta 6060
   cctgtcaggc cgagccctgc tggcttcgca cttaacatct ccaggtctca gtatcacttc 6120
   ctgctactta gcacagttag gagttgagca aacctttttt tccaaccccc actaaaattt 6180
   aattgacaaa agactgtgta atttgtggga tacagtgtga taattgatct atgtgtgcat 6240
   tgtgcaaggt tcaataagat agattaatag gcccatcaac agctttatgg gtgtgaaatg 6300
   caagtaatat aggtagatgc ctgtggtgtc cttaggtcag aaaggcatga ttttaaggtc 6360
   ttgggcaaat catattatac tcatgctaaa aatacattat gttgattatt aatcttttag 6420
   agaaggctga tacttggttt tggtgctcag caagcaaatg tcaccagctc tttctaactg 6480
   gtaccacttt agaaaatgct acctgtgctc aaattggttt gtattcttat tttcatagct 6540
   tggagagagt ggagagatca aggcgattgg ggaactggac ctgctgttta tgtctctgag 6600
   aaatgcttgc gtctgagcga gaagaagcta gaaaacgaag aactgctcct tcctgccttc 6660
   taaaaagaac aataagatcc ctgaatggac ttttttacta aaggaaagtg agaagctaac 6720
   gtccatcatc attagaagat ttcacatgaa acctggctca gttgaaaaag aaaatagtgt 6780
   caagttgtcc atgagaccag aggtagactt gataaccaca aagattcatt gacaatattt 6840
   tattgtcact gatgatacaa cagaaaaata atgtacttta aaaaattgtt tgaaaggagg 6900
   ttacctctca ttcctttaga aaaaaagctt atgtaacttc atttccatat ccaatatttt 6960
   atatatgtaa gtttatttat tataagtata cattttattt atgtcagttt attaatatgg 7020
   atttatttat agaaacatta tctgctattg atatttagta taaggcaaat aatatttatg 7080
   acaataacta tggaaacaag atatcttagg ctttaataaa cacatggata tcataaatct 7140
   tctgtcttgt aatttttctc cctttaatat caacaatacc atcatcatca tcattaccca 7200
   atcattctca tgatttcatg cttgacccat attatactgt taaagttggt tcctggaggc 7260
   ctgtggtttt gtgtgtgttg tgtgtgtgtg tggggttatg catgtgaaag ccagagatgg 7320
   atattaggtg ttcttctcta tcagtctttg ccttattatt tgagacaggg tctgtcactg 7380
   aacctgtagc taggctggcc aacaagctct attaattttt tttaagatta attaattatg 7440
   tgtat 7445
<210> 9
   <211> 1111
   <212> DNA
   <213> Mus musculus
   <220>
   <400> 9
<210> 10
   <211> 21
   <212> DNA
   <213> Mus musculus
   <220>
   <400> 10
   ctgcctgctt ctcattgccc t 21
<210> 11
   <211> 21
   <212> DNA
   <213> Mus musculus
   <220>
   <400> 11
   caagtctacc tctggtctca t 21
<210> 12
   <211> 20
   <212> DNA
   <213> Mus musculus
   <220>
   <400> 12
   gacgcaagca tttctcagag 20
<210> 13
   <211> 16
   <212> DNA
   <213> Homo sapiens
   <220>
   <400> 13
   atgtatttcc cagaaa 16
<210> 14
   <211> 17
   <212> DNA
   <213> Homo sapiens
   <220>
   <400> 14
   ccttttctgg gaaatac 17
<210> 15
   <211> 22
   <212> DNA
   <213> Mus musculus
   <220>
   <400> 15
   aggtgctcaa cttcaccctg ga 22
<210> 16
   <211> 22
   <212> DNA
   <213> Mus musculus
   <220>
   <400> 16
   ccactctctc caagcttttt ca 22
<210> 17
   <211> 21
   <212> DNA
   <213> Mus musculus
   <220>
   <400> 17
   caagtctacc tctggtctca t 21
<210> 18
   <211> 418
   <212> DNA
   <213> Homo sapiens
   <220>
   <400> 18
   agaagtgctg ttccctcaat ctgataggtt ccagccttat atgcaggagg tggtgccctt 60
   cctggccagg ctcagcaaca ggctaagcac atgtcatatt gaaggtgatg acctgcatat 120
   ccagaggaat gtgcaaaagc tgaaggacac agtgaaaaag cttggagaga gtggagagat 180
   caaagcaatt ggagaactgg atttgctgtt tatgtctctg agaaatgcct gcatttgacc 240
   agagcaaagc tgaaaaatga ataactaacc ccctttccct gctagaaata acaattagat 300
   gccccaaagc gatttttttt aaccaaaagg aagatgggaa gccaaactcc atcatgatgg 360
   gtggattcca aatgaacccc tgcgttagtt acaaaggaaa ccaatgccac ttttgttt 418
<210> 19
   <211> 21
   <212> DNA
   <213> Homo sapiens
   <220>
   <400> 19
   tggccaggaa gggcaccacc t 21
<210> 20
   <211> 21
   <212> DNA
   <213> Homo sapiens
   <220>
   <400> 20
   cctatcagat tgagggaaca g 21
<210> 21
   <211> 36
   <212> DNA
   <213> artificial sequence
   <220>
   <223> Description of Artificial Sequence PCR primer
   <220>
   <221> modified_base
   <222> 24,25,29,30,34,35
   <223> a is inosine
   <400> 21
   ggccacgcgt cgactagtac gggaagggaa gggaag 36
<210> 22
   <211> 20
   <212> DNA
   <213> artificial sequence
   <220>
   <400> 22
   ggccacgcgt cgactagtac 20
<210> 23
   <211> 20
   <212> DNA
   <213> Homo sapiens
   <220>
   <400> 23
   ccttccccag tcaccagttg 20
<210> 24
   <211> 20
   <212> DNA
   <213> Homo sapiens
   <220>
   <400> 24
   taattgttat tcttagcagg 20
<210> 25
   <211> 690
   <212> DNA
   <213> Homo sapiens
   <220>
   <400> 25
   tgcacaagca gaatcttcag aacaggttct ccttccccag tcaccagttg ctcgagttag   60
   aattgtctgc aatggccgcc ctgcagaaat ctgtgagctc tttccttatg gggaccctgg  120
   ccaccagctg cctccttctc ttggccctct tggtacaggg aggagcagct gcgcccatca  180
   gctcccactg caggcttgac aagtccaact tccagcagcc ctatatcacc aaccgcacct  240
   tcatgctggc taaggaggct agcttggctg ataacaacac agacgttcgt ctcattgggg  300
   agaaactgtt ccacggagtc agtatgagtg agcgctgcta tctgatgaag caggtgctga  360
   acttcaccct tgaagaagtg ctgttccctc aatctgatag gttccagcct tatatgcagg  420
   aggtggtgcc cttcctggcc aggctcagca acaggctaag cacatgtcat attgaaggtg  480
   atgacctgca tatccagagg aatgtgcaaa agctgaagga cacagtgaaa aagcttggag  540
   agagtggaga gatcaaagca attggagaac tggatttgct gtttatgtct ctgagaaatg  600
   cctgcatttg accagagcaa agctgaaaaa tgaataacta accccctttc  cctgctagaa  660
   ataacaatta gatgccccaa agcgattttt                                   690
<210> 26
   <211> 4797
   <212> DNA
   <213> Homo sapiens
   <220>
   <400> 26
   tgcacaagca gaatcttcag aacaggttct ccttccccag tcaccagttg ctcgagttag   60
   aattgtctgc aatggccgcc ctgcagaaat ctgtgagctc tttccttatg gggaccctgg  120
   ccaccagctg cctccttctc ttggccctct tggtacaggg aggagcagct gcgcccatca  180
   gctcccactg caggcttgac aagtccaact tccagcagcc ctatatcacc aaccgcacct  240
   tcatgctggc taaggaggta tacatctcaa tcctgctctt tctcgttgga tctacttgga  300
   atccaaatag ttcttaaact tttcttcaga gcatctctaa gagctttagg aacccactgt  360
   ttatccctga gggtagataa attttctgtt  ttttcagaga ctctttggga atctggcttt  420
   ttttttttct tgaacttctt ccttccattt tggcctttat gatacatatg atgaattttt 480
   cccaaagagc ggccattcag taatccatct gatgattttt ttttccttta tgcctctgtg 540
   cattgttcta aactcatgca cacatctgaa ttctgctttt agtctttatg atgttgctct 600
   ggggagacgg gatggggcac atgtctatgt ataaattttt tttctatttg ctcaatgtcc 660
   agacccttag tcttttcttc tcttccaggc tagcttggct gataacaaca cagacgttcg 720
   tctcattggg gagaaactgt tccacggagt cagtgtaagc tacagttgtg acgaacaggg 780
   ccgtgtgccg tccatgggta cttggggtgg tggtgatgat ggtttaggtc ttatccctta 840
   tgaccctttc tgtttccctt ccacctgcag atgagtgagc gctgctatct gatgaagcag 900
   gtgctgaact tcacccttga agaagtgctg ttccctcaat ctgataggtt ccagccttat 960
   atgcaggagg tggtgccctt cctggccagg ctcagcaaca ggctaagcac atgtgtaagt 1020
   tcagctctca gcctatgccc acctacccct ccttccctcc ttccacagag acccccttac 1080
   cccaactctc tctccttccc cctaccccta agctagcagg aagaagtgtc ttggcagcag 1140
   tgttatcagg agtcatttgg gatcatagag tatttgcttt tgctttgact gagtcacatc 1200
   ttgagtttat agtggtgaat ggggtctgga acttaagtgt acagaagccg cattggtttg 1260
   tcttcggaaa aaaggcaact caggttgcgt aagatgagaa aggtgttggg aaaacatcta 1320
   gctgtggaaa tggatccatt gagtctaagt tgttgagggg aggggatggc atggagagaa 1380
   attagaagag aaagtgggaa atgggaaggc ttaaagtcgg tggtgggtcg gcagactgtt 1440
   gccctgttga tgtcatggga agccacaaaa tcggaggcgt gtgaacttga tgccgctgaa 1500
   catttgaaac tatgaaaaaa agtttgagtg gagtgggccc agtaaaaggc cctaggactt 1560
   actgaagagg gcttaatttt cacatgagat gttttatgta catttcttgt tctaagcatg 1620
   caattttctg gagatacgat tgaggtttta ttccttacag aatttgcata aactactccg 1680
   ctctttccac aaatgcaaac ctcagtagga tttcccaaag atgaagagag gtctcttgta 1740
   agggaagtga ctggattctg gcgtccaagg gaattcaaga gctcaggaaa tctaggtcac 1800
   tgttgaaatc taggtcattg tgggcaaaat tactaagagc tttaattcca ggtgaattgt 1860
   actgtacctc catgggtgtg gaggttcata aagtttcagc acaacattaa gatagttatg 1920
   cttgttattg ttttatagca tattgaaggt gatgacctgc atatccagag gaatgtgcaa 1980
   aagctgaagg acacagtgaa aaaggtagga ctgataactg tcaatgctaa gtcatgcaat 2040
   aggagagaca aatgttgttt ttctttcctt tctttcttcc catcactttg tgatttttca 2100
   cttgattctc ctaccaccag ggcgattact ttggtgtctg tgtatgtaga tatatctata 2160
   tatctagatg tcagtttcca aatcttgcaa attgtagaat tctagaactg gttgggatct 2220
   tagcttgtct agtcacataa cctcagattc tggggatggt cagtggcaga gatagggcta 2280
   gaatgcaggt ctcctgaatc ccaagccagc acttttcccg gtggtgatac agattagttt 2340
   tggtaccatt aattcttagg gaaatttcag attcctattg actcatgtaa tctgaagaag 2400
   tacttgttta aaaacagaaa aatgcctatg ggcaaattta tttgaagtca tttttgaagt 2460
   cattaatgca ttgctttgaa acttggaaga ataaactcag aacaatgaga aaagagctgg 2520
   acttgcatat agggctaatt tctggagtaa taaacactta ttttgaatta tcataatatc 2580
   tatcagatat tgattatagt ttaaaagcaa gagcagacaa ccccgatctc ttttatacag 2640
   gttcaaatag agtaaaaata ttagtaagag atttattata gttaaatgga agtctgaatt 2700
   ggtaagcttt tttttcttcc tctctcccat caagaccttc cattctagtt tcttccttca 2760
   ctccctcaac aaatccctag ggagcattta tccatggtgg gctggtgtac atttctatag 2820
   tgaatgatac catcatgtgg cctatttggt gaaaagaaca acaatggaag gcttagacta 2880
   acaatagtga ctcaccccaa aaccggagga atgattagga gcagtgaaag tgacgctctt 2940
   gcaagcaggt acaactaaat actcagaaac atgaaggctc cagttgatgg aattttcagt 3000
   aacaagctta accttaattc cccctttttc cctcttgact ttttaaaaaa gcgtttcttc 3060
   ctgagcatca tttaatgagt gtgactgttt cttcctttga taattgaagg ctttgtagtt 3120
   ttaaattgtg aagcccagtt ctcttgttat agaactatta tctagacatg gagggctgaa 3180
   tgttagcatg ccacagacaa ggcatgcttt acacatcttg cttaaaaaat tactgatttc 3240
   atcttgcttg ttgtctttag aaaagtgaag tgtgagagag gagaatctca tggtgatctg 3300
   tgtgattttc aagaccttta atccattttg aaagaatcaa tttcatattt gcaatgggtt 3360
   gccatgtgga agagtgatta tgcttttttg ctggtagctt cagaaagcac aggagggaga 3420
   gcaatgttgt tcagagaaag atcaacagga ggagaaactg tcagagctgt ctgaaatagg 3480
   gtggttttgg gaggcattaa ttccctctcg ttgggggtaa aagcagaacg caggttggta 3540
   gtaaaatgca tgacagacag taggggacga taaactttaa aattctttat agtcttggag 3600
   tctttgagat agaaaagaat atctttttgg ccttatgtca aaagaagtat ggaaaggtga 3660
   aagggcggaa gaaagcagga aaaggaagaa ccatgtatta tatagaggac aatggtgaca 3720
   aggtttttct tgaaataatg caaatatgat agattagagg aatttcagta gggaatgctt 3780
   ttcacttgaa tttgggtttc ctcttcgatt aagtttggga tcctcatctg catttgactt 3840
   ggagagagaa agaatgaatg ttaggaccta tatctggttt tctattaact aaagcaagtg 3900
   gaaaagactt atttggtatt tttcccacaa aagtgaaaac ttttctttta ctgtttgtca 3960
   aaaaggtgga aatagaaaaa gccttaatgt attggtgaat acatggttca aagtcatttg 4020
   agtagagatg ttttaaatca ggagtgtcca atcatttggc ttccctggac caccttgaaa 4080
   gaattgtctt ggtacacaca taaaatacaa gaacaatagc tgatgagcta aaaaagtcca 4140
   tgcataaatc tcatactgtt ttaagaaagt ttatgaattt ctgttagggt gcattcaaag 4200
   ctgtcctggg ccatgtgcgg cctgtgggct gcaggttgga caagctcctt ataagtaatc 4260
   tgtcatagat agttttggag ctgcaaaaca ggccaaggca taatgggtgg cactcgggat 4320
   cccccagatc ccagcctcac ttcagtctcc ttgctctggt taagaagggg tggtcaactc 4380
   tctgcccagc ttttaaacag cttcattagt gtgaggtgca cctgaaattg atgcctgctg 4440
   gtggcctctc agtccagaga gccgtcattt taagctcttt ggcaaatcat acaatactaa 4500
   agggatatta ctatgaatgt tttacaaatg cttaaaactc ggtttctgtc tccatcaacc 4560
   taatcttgca atttctaatt tgttcacttt agaaaacatg gcataaatgc tcaaatactt 4620
   ttgcattctt attttcacag cttggagaga gtggagagat caaagcaatt ggagaactgg 4680
   atttgctgtt tatgtctctg agaaatgcct gcatttgacc agagcaaagc tgaaaaatga 4740
   ataactaacc ccctttccct gctagaaata acaattagat gccccaaagc gattttt     4797
<210> 27
   <211> 20
   <212> DNA
   <213> Homo sapiens
   <220>
   <400> 27
   atcagatgga ttactgaatg 20
<210> 28
   <211> 20
   <212> DNA
   <213> Homo sapiens
   <220>
   <400> 28
   agctcagcta cagcacagat 20
<210> 29
   <211> 20
   <212> DNA
   <213> Homo sapiens
   <220>
   <400> 29
   cctgccccat ttattggcag 20
<210> 30
   <211> 20
   <212> DNA
   <213> Homo sapiens
   <220>
   <400> 30
   tgtcctctgc caccctaaca 20
<210> 31
   <211> 20
   <212> DNA
   <213> Homo sapiens
   <220>
   <400> 31
   taattcacca ggaccatcat 20
<210> 32
   <211> 20
   <212> DNA
   <213> Homo sapiens
   <220>
   <400> 32
   gtggactcag gcaatgatgt 20
<210> 33
   <211> 20
   <212> DNA
   <213> Homo sapiens
   <220>
   <400> 33
   acatagagtg ttaaagtggg 20
<210> 34
   <211> 20
   <212> DNA
   <213> Homo sapiens
   <220>
   <400> 34
   gctggaaggt ggacagcgag 20
<210> 35
   <211> 20
   <212> DNA
   <213> Homo sapiens
   <220>
   <400> 35
   tggcatcgtg atggactccg 20
<210> 36
   <211> 20
   <212> DNA
   <213> Mus musculus
   <220>
   <400> 36
   tctgctccct gctcctggga 20
<210> 37
   <211> 20
   <212> DNA
   <213> Mus musculus
   <220>
   <400> 37
   tccaggaggt ctgtagtaat 20
<210> 38
   <211> 21
   <212> DNA
   <213> Mus musculus
   <220>
   <400> 38
   ctgcctgctt ctcattgccc t 21
<210> 39
   <211> 21
   <212> DNA
   <213> Mus musculus
   <220>
   <400> 39
   caagtctacc tctggtctca t 21
<210> 40
   <211> 179
   <212> PRT
   <213> Mus musculus
   <220>
   <400> 40
   Met Ala Val Leu Gln Lys Ser Met Ser Phe Ser Leu Met Gly Thr Leu
   1               5                   10                  15
   Ala Ala Ser Cys Leu Leu Leu Ile Ala Leu Trp Ala Gln Glu Ala Asn
               20                  25                  30
   Ala Leu Pro Val Asn Thr Arg Cys Lys Leu Glu Val Ser Asn Phe Gln
           35                  40                  45
   Gln Pro Tyr Ile Val Asn Arg Thr Phe Met Leu Ala Lys Glu Ala Ser
       50                  55                  60
   Leu Ala Asp Asn Asn Thr Asp Val Arg Leu Ile Gly Glu Lys Leu Phe
   65                  70                  75                  80
   Arg Gly Val Ser Ala Lys Asp Gln Cys Tyr Leu Met Lys Gln Val Leu
                   85                  90                  95
   Asn Phe Thr Leu Glu Asp Val Leu Leu Pro Gln Ser Asp Arg Phe Gln
               100                 105                 110
   Pro Tyr Met Gln Glu Val Val Pro Phe Leu Thr Lys Leu Ser Asn Gln
           115                 120                 125
   Leu Ser Ser Cys His Ile Ser Gly Asp Asp Gln Asn Ile Gln Lys Asn
       130                 135                 140
   Val Arg Arg Leu Lys Glu Thr Val Lys Lys Leu Gly Glu Ser Gly Glu
   145                 150                 155                 160
   Ile Lys Ala Ile Gly Glu Leu Asp Leu Leu Phe Met Ser Leu Arg Asn
                   165                 170                 175
   Ala Cys Val
<210> 41
   <211> 179
   <212> PRT
   <213> Mus musculus
   <220>
   <400> 41
   Met Ala Val Leu Gln Lys Ser Met Ser Phe Ser Leu Met Gly Thr Leu
   1               5                   10                  15
   Ala Ala Ser Cys Leu Leu Leu Ile Ala Leu Trp Ala Gln Glu Ala Asn
               20                  25                  30
   Ala Leu Pro Ile Asn Thr Arg Cys Lys Leu Glu Val Ser Asn Phe Gln
           35                  40                  45
   Gln Pro Tyr Ile Val Asn Arg Thr Phe Met Leu Ala Lys Glu Ala Ser
       50                  55                  60
   Leu Ala Asp Asn Asn Thr Asp Val Arg Leu Ile Gly Glu Lys Leu Phe
   65                  70                  75                  80
   Arg Gly Val Ser Ala Lys Asp Gln Cys Tyr Leu Met Lys Gln Val Leu
                   85                  90                  95
   Asn Phe Thr Leu Glu Asp Ile Leu Leu Pro Gln Ser Asp Arg Phe Arg
               100                 105                 110
   Pro Tyr Met Gln Glu Val Val Pro Phe Leu Thr Lys Leu Ser Asn Gln
           115                 120                 125
   Leu Ser Ser Cys His Ile Ser Gly Asp Asp Gln Asn Ile Gln Lys Asn
       130                 135                 140
   Val Arg Arg Leu Lys Glu Thr Val Lys Lys Leu Gly Glu Ser Gly Glu
   145                 150                 155                 160
   Ile Lys Ala Ile Gly Glu Leu Asp Leu Leu Phe Met Ser Leu Arg Asn
                   165                 170                 175
   Ala Cys Val
<210> 42
   <211> 5935
   <212> DNA
   <213> Mus musculus
   <220>
   <400> 42
   gaattcaagt ccacatgcaa tcaatccgaa tactttgtaa attctcttct tcaaatatcc   60
   atctatatag tataagttat tgtaggatca tttaaaaata atgttttgag acttatgttt  120
   gcacaagtaa aatgtcagag agaattagca aatgtatagt attattttat tttaaaaaat  180
   ctatgcttaa aatgtctatt agattgttca ctactgacat ttccaaactt aacttgacct  240
   tggctatgat ttcaaccttt gtatttgcat ctaccataac tgtgtgctca cttaccatgc  300
   tatccgacga gcatgttccc ctgatgtttt tgccttttgc tctctcgcta acaggctctc  360
   ctctcagtta tcaacttttg acacttgtgc gatcggtgat ggctgtcctg cagaaatcta  420
   tgagtttttc ccttatgggg actttggccg ccagctgcct gcttctcatt gccctgtggg  480
   cccaggaggc aaatgcgctg cccatcaaca cccggtgcaa gcttgaggtg tccaacttcc  540
   agcagccgta catcgtcaac cgcaccttta tgctggccaa ggaggtacag ctgcatctct  600
   ttctctccat accgccttgc catttctctg aagcacttgc aaactcttta ggggcgcttt  660
   atctccgcag gtctcactac ctatgttttc tgtctcttta gagactcttt aaggactgga  720
   tctttttcta tttctatttc aaggtctcag gaccatttcc tatcttggcc ttcaggacac  780
   atatactgaa ttttatctac agaggcgcgt ttagaaagcc acccacgact gcaatacttt  840
   ccatcctgtt gtgctctctt ctgaactcat actctcttgg ctactcctga gacccactgc  900
   ggacatacat ctctacttac aggcttttct tccatctcct tgtcacccag gcacttaggg  960
   ttttctctct ttcaggccag ccttgcagat aacaacacag acgtccggct catcggggag 1020
   aaactgttcc gaggagtcag tgtaagtcct cactgtgatg agcagggcta gctgcgggag 1080
   ctggtggacc ctctgggata gtctgacgta tgacccctgc tgcttcttgt ctacctgcag 1140
   gctaaggatc agtgctacct gatgaagcag gtgctcaact tcaccctgga agacattctg 1200
   ctcccccagt cagacaggtt ccggccctac atgcaggagg tggtgccttt cctgaccaaa 1260
   ctcagcaatc agctcagctc ctgtgtaagt ctggctctgg ctacctatgc tcctctctct 1320
   tcctcttcta ttccagtaag aacccgaggt cctgccctct ctctcttcac aagagtgagg 1380
   agggcctcag caccaccacc atcataggcc acttgaaata ggtcacaaag gctttggctt 1440
   caattgagta atactttgag tttgtattag ttaagcttta tttgttttat ccatggaaag 1500
   aaatcaactc aaattctgta ggatgagaaa gatgttggga acgaaaaaag gcctagatag 1560
   agaaacagat ctgctgagta cagtacttat gggggggggg ggcagggggc gatatccact 1620
   gagtccaagt acttgttggg agagaaatcc actgagtaca agtacttgtg ggggaaggaa 1680
   tggcacagag caaaagttga agggaaagag gaagatggag aggcctcaat gttgggggtg 1740
   tgaaaggtca ctcctttttc catgtgatgg agagttaaga aaaatcagtg tgtgagtttg 1800
   atgtcttcag acaccccaac tatggcagac tgtgggagac ctggcattta gggaaggcgc 1860
   ggcttttcac acgagaaact ttatgctcat ctcttgtgct acactcccac ctttgatgag 1920
   gttaagctca ggtttcgttt ctaccgttct tgctactggt ggaaacttca gtaggattcc 1980
   ccaaagacga ggacagctct tctgtaaggg agggacctgg atttcagtgt cctagagaac 2040
   gaaatagctc agagaatcta ggtcaacgtg aaatctaggt cacagcgggc aaaaatgact 2100
   gaacgcctct attccaggtg aacggtcacg tgcctcagat atactgaggt attgggctcc 2160
   caccggataa gattctgtta gtgagtctgc ttttattttg cagcacatca gtggtgacga 2220
   ccagaacatc cagaagaatg tcagaaggct gaaggagaca gtgaaaaagg tactattggc 2280
   aagccacaat actaagccat tcagtaggag acgtggggat ttctttctct gcttcccagt 2340
   ctcttctact ttgtaacatt ttctttgact tgtctactgt ctggtccatt actcacttag 2400
   ctgcacctgc atctagctgg gtctatagat ctttcaatct gtgtctaaat ttgtaagtca 2460
   caattctgga gctagcagaa agcttagctc agccagtctc atgagcactt gctcggagga 2520
   tggcttgtga cagagtcaat gctagaagac agcatccctg attcccagct ctgcacttgc 2580
   ctagtggcca cgtgtaatta ctttagcctg attaagtatt tgggaaagcc aattcccacc 2640
   gacctacata atccgaagaa gcatgcattg aaaactagaa agctgggcac aaacttacta 2700
   gagatgattt ttgagctcat taaactgatg ctctgaaatg tgatcaaatc aacccagaat 2760
   aacaacaaaa gagctggatt tgcaaatagg acaagtattt agaatcactg gtattaacag 2820
   ctgtcatctt aattaaaata tagtgtctat ttagctgcct atttaagatt aaacacaaga 2880
   gtggataact tcccaattta ctgggcctgg tttcaataga gtaaaaatat cagtcataga 2940
   ttaattatag tgtcatgaaa gtatgagttg gaaacccttt ccttactttt taccttcatt 3000
   tcttagttat tatttttttt tcttcacacc ctgatcaagc cactagtaag cacctatctg 3060
   ctgcgagcta ttatatgact ttacagcaaa caacattgct gtgtggcctc tttggggaag 3120
   ggaacaggat agcaggaggc tcaggctagc aagtctggac tcaacctaaa gccagaggca 3180
   tggttgatag cagagaaagt gaggctcttc acaagtgggt gtgcttaagt aatcagaaac 3240
   aggaaggctc tggttgatgg aattatcagt aagatatcta cccttatctc cttcttctat 3300
   agaagctaaa ccgtctctcc ttcttgtgtg taggctgata aacacgcttg ttttcttttg 3360
   agtgttcatg gctttgcaga ttttcagtgc tctgccagtt cttgttagag ggtttgttac 3420
   cttgacacct gggcttggat gttagcatgc caaaggcaca cacttctgaa tgcctgtgta 3480
   aaaggttatt attcatttac tttgtctttg gaaaggtgaa gtgtgtgtga gaaagaactc 3540
   acaggagatg tattctctgt aggaaaactt ttttttcccc ttaaaagcct ataatccact 3600
   ttcagtcaac tttgactttt ataccatgct gtcacatgaa agagtgttta ggcccgctct 3660
   cgtggctctg ggaaaagcac caatagggga agaaatgtta tgccgagaaa tctgactggc 3720
   agggaaactg ggtcagagct ccccaaagac cactacaggt gttaagtagg aacagtcgag 3780
   ggtgggttca tataatagaa tggaacagag ggagggaaga taagctacaa agtttcatag 3840
   ggtcctaagt ctttaagata caaaatagct ggttgggctt cataacaaag gaagtctggg 3900
   aaggcagcaa gcattgagag ggagatggaa agggaaaaaa caatgtagag gatttgaaaa 3960
   gctacaaatc ctccacgaga ggatttttct tggaggaatc tagaacaagg gtggtggatt 4020
   aggtggatcg cagaaggact tgctttgcca tttgaatctg ggtttttgtc tctccattga 4080
   ggttgagagc gtcacccttt tttaccctgg ataggaggag gaaagaaggg gtgttttgac 4140
   tcctacctgg agttttacta gtttacgcaa tggaacagac actcgggacc tcctcttgac 4200
   aagaaaaaaa aaaaaaaaag gaaacctgtt gtttctcttg tttgttcttt tgttaagaaa 4260
   gcacaggcag ctgggcatgg tggcccatgc ctttaatccc agcatttggg aggcagaggc 4320
   aggtgacttt ctaaattcaa ggccagcctg gtctacaaag tgagttccag gacagccagg 4380
   gctatacaga gaaaccctgt ctcgggaaaa aaaaaaaaga agaaaagaaa agaaaagaag 4440
   agaagaggag aggagaggag aggagaggag aggagaggag aggagaggag aggagaggag 4500
   aggagaggag aagagaagag aagagaagag aagagaagag aagagaagag aagagaagag 4560
   aagagaagag aagagaagag aagagaagag aagagaagag aagagaaaag aaaagagaaa 4620
   agaaaagaaa aaagcaagca agcaagcact ggcaaagcat gcccacatgg gacgtatgtg 4680
   ggtctttgag acaaggcttt tgaattgagc gctcatcaat agttgatcat ggtcaggtgg 4740
   agggctacct gtcaggccga gccctgctgg cttagcactt aacatctcca ggtctcagta 4800
   tcacttcctg ctgcttagca cagttaggag ttgagcaaac ctttttttcc aacccccact 4860
   aaaatttaat ttacaaaagg cagtgtaatt tgtgggatac agtgtgataa ttgatctatg 4920
   tgtgcattgt gcaaggttca ataaggtaga tcaataggcc catcaacagc tttatgggtg 4980
   tgaaatgcaa gtaatatagg tagatgcctg tgtgtcctta ggtcagaaag gcatgatttt 5040
   aaggtcttgg gcaaatcata ttatactcat gttaaaaatg cattatgttg attatcaatc 5100
   ttttagagaa ggctgatact tggttttggt gctcagcaag caaatgtcac cagctctttc 5160
   taactagtac cactttagaa aatgctaccc gtgctcaaat tggtttgtat tcttattttc 5220
   atagcttgga gagagcggag agatcaaagc gatcggggaa ctggacctgc tgtttatgtc 5280
   tctgagaaat gcttgcgtct gagcgagaag aagctagaaa acgaagaact gctccttcct 5340
   gccttctaaa aagaacaata agatccctga atggactttt ttactaaagg aaagtgagaa 5400
   gctaacgtcc accatcatta gaagatttca catgaaacct ggctcagttg aaagagaaaa 5460
   tagtgtcaag ttgtccatga gaccagaggt agacttgata accacaaaga ttcattgaca 5520
   atattttatt gtcattgata atgcaacaga aaaagtatgt actttaaaaa attgtttgaa 5580
   aggaggttac ctctcattcc tctagaagaa aagcctatgt aacttcattt ccataaccaa 5640
   tactttatat atgtaagttt atttattata agtatacatt ttatttatgt cagtttatta 5700
   atatggattt atttatagaa aaattatctg atgttgatat ttgagtataa agcaaataat 5760
   atttatgata ataactatag aaacaagata tcttaggctt taataaacac atgaatatca 5820
   taaatcttct gtcttgtaat ttttctccct ttaatatcaa caataccatc atcgtcatca 5880
   ttacccaatc attctcatga cttcatgctt gactcatatt atctggtaaa gtttg 5935
<210> 43
   <211> 179
   <212> PRT
   <213> Homo sapiens
   <220>
   <400> 43
   Met Ala Ala Leu Gln Lys Ser Val Ser Ser Phe Leu Met Gly Thr Leu
   1               5                   10                  15
   Ala Thr Ser Cys Leu Leu Leu Leu Ala Leu Leu Val Gln Gly Gly Ala
               20                  25                  30
   Ala Ala Pro Ile Ser Ser His Cys Arg Leu Asp Lys Ser Asn Phe Gln
           35                  40                  45
   Gln Pro Tyr Ile Thr Asn Arg Thr Phe Met Leu Ala Lys Glu Ala Ser
       50                  55                  60
   Leu Ala Asp Asn Asn Thr Asp Val Arg Leu Ile Gly Glu Lys Leu Phe
   65                  70                  75                  80
   His Gly Val Ser Met Ser Glu Arg Cys Tyr Leu Met Lys Gln Val Leu
                   85                  90                  95
   Asn Phe Thr Leu Glu Glu Val Leu Phe Pro Gln Ser Asp Arg Phe Gln
               100                 105                 110
   Pro Tyr Met Gln Glu Val Val Pro Phe Leu Ala Arg Leu Ser Asn Arg
           115                 120                 125
   Leu Ser Thr Cys His Ile Glu Gly Asp Asp Leu His Ile Gln Arg Asn
       130                 135                 140
   Val Gln Lys Leu Lys Asp Thr Val Lys Lys Leu Gly Glu Ser Gly Glu
   145                 150                 155                 160
   Ile Lys Ala Ile Gly Glu Leu Asp Leu Leu Phe Met Ser Leu Arg Asn
                   165                 170                 175
   Ala Cys Ile